(19) Europäisches Patentamt
European Patent Office
Office européen des brevets

(11) **EP 3 154 520 B1**

(12) **EUROPEAN PATENT SPECIFICATION**

(45) Date of publication and mention
of the grant of the patent:
**29.04.2020  Bulletin 2020/18**

(51) Int Cl.:
***A61K 9/127*** *(2006.01)*    ***A61K 38/27*** *(2006.01)*

(21) Application number: **15731249.7**

(86) International application number:
**PCT/EP2015/001192**

(22) Date of filing: **12.06.2015**

(87) International publication number:
**WO 2015/188946 (17.12.2015 Gazette 2015/50)**

(54) **MATRIX STABILIZED LIPOSOMES**

MATRIXSTABILISIERTE LIPOSOMEN

LIPOSOMES STABILISÉS PAR MATRICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.06.2014  DE 102014008657**

(43) Date of publication of application:
**19.04.2017  Bulletin 2017/16**

(73) Proprietor: **HEIDELTEC GmbH**
**69120 Heidelberg (DE)**

(72) Inventors:
• **PARMENTIER, Johannes**
  **60329 Frankfurt am Main (DE)**
• **PANTZE, Silvia**
  **69120 Heidelberg (DE)**
• **FRICKER, Gert**
  **69221 Dossenheim (DE)**

(74) Representative: **Müller-Boré & Partner**
**Patentanwälte PartG mbB**
**Friedenheimer Brücke 21**
**80639 München (DE)**

(56) References cited:
**US-A1- 2002 146 447    US-A1- 2011 020 428**

## Description

The present invention relates to a solid dosage form comprising liposomes, water in an amount of between 5% and 95% (w/w), based on the total mass of the solid dosage form, and one or more solidifier(s), wherein said solidifier(s) form(s) a solid matrix in which said liposomes are embedded, and is/are contained in the inner lumen of said liposomes. The present invention further relates to a method for the production of respective solid dosage forms.

Oral drug delivery is considered as the most advantageous way of application, in particular for the treatment of chronic diseases, which demand long-term and repeated drug administration. The oral route offers high drug safety and is widely accepted among patients due to its convenience. Additionally, non-sterility of oral drug forms reduces costs in production, storage and distribution, which could contribute to health care improvement in third world countries. It is estimated, that 90% of all marketed drug formulations are for oral use. However, not all drugs are suitable for the oral route, because of low solubility in the gastrointestinal tract (GIT), poor stability or low permeation across the intestinal mucosa or a combination of both.

Every drug has to overcome several physico-chemical and metabolic constraints before it can reach the blood system (G. Fricker et al., J. Pept. Sci., 2 (4), 195-211, 1996). After oral administration drug forms pass in the following order: mouth, pharynx, esophagus, stomach, duodenum, jejunum, ileum and colon. Saliva in the mouth contains amylase, lysozyme and mucus. Absorption of most drugs is low due to the short retention time in the mouth and the low permeation across the oral mucosa (H. Sohi et al., Drug. Dev. Ind. Pharm., 36 (3), 254-282, 2010). Pharynx and esophagus play only a minor role in oral drug application since food and drug formulations pass in usually less than 10 seconds (R. Singh et al., J. Pharm. Sci., 97 (7), 2497-2523, 2008). The two main functions of the stomach are food storage and digestion, whereas no food absorption takes place and drugs are only absorbed to a low extent. Hydrochloric acid and pepsinogen, which is quickly converted to the proteolytic enzyme pepsin in the stomach, are secreted to break down food proteins, but also therapeutic proteins can be degraded. Acidic conditions in the stomach can increase solubility of basic drugs, but can be harmful to peptides and other acid-labile drugs. The small intestine is the major absorption organ of the GIT due to its large surface area of about 200 m$^2$, active transport processes and less pronounced mucus layer compared to the stomach. In the duodenum, pancreatic and biliary juices are released in the lumen for further food digestion and to facilitate uptake of nutrients (M. Shimizu, Nahrung 43 (3), 154-158, 1999). The pancreas produces different amylases, proteases and lipases, which eventually break down food components to oligo- and disaccharides, oligopeptides and glycerol and free fatty acids. Latter are finally emulsified by different bile salts and can be taken up as mixed micelles. Luminal secretions have a strong influence on the fate of orally administered drugs and drug forms, too. Pancreatic enzymes can degrade lipid and protein based drug carriers, peptide and other drugs and bile salts might destabilize lipid drug carriers, such as liposomes, but can also improve the solubility and bioavailability of poorly soluble drugs. Final digestion of food components to amino acids and monosaccharides is performed by enzymes expressed in the brush border of enterocytes, e.g. glycosidases and peptidases. Furthermore, active and passive uptake transporters for nutrients, but also transporters for the efflux of xenobiotic compounds, *e.g.* P-glycoprotein, can be found in the luminal membrane of enterocytes. Multidrug resistance proteins can reduce the bioavailability of many different drug types, whereas the contribution of active uptake mechanisms in oral drug delivery remains unclear. Moreover, the intestinal epithelium is covered with mucus, which acts as a mechanical protection against microbiological and chemical pathogens (J. Hamman et al., Bio-Drugs, 19 (3), 165-177, 2006). Enterocytes are organized in a dense monolayer and the interstitial space between cells is closed by tight junctions allowing only the permeation of ions but not of macromolecules (V. Tang et al., Biophys. J. 84 (3), 1660-1673, 2003). The colon is the last part of the GIT and here mainly water and salt absorption takes place, whereas only a few drugs and nutrients, *e.g.* fat soluble vitamins, are absorbed. Due to its low activity of digestive enzymes it has gained increasing importance in delivery of unstable drugs, such as proteins and peptides.

Macromolecules, in particular peptides, polypeptides and proteins as well as small molecules play an important role in genesis and therapy of many diseases (G. Fricker et al., J. Pept. Sci., 2 (4), 195-211, 1996). Endogenous peptides take part in the control of almost all body functions and many protein drugs are used to treat severe and often chronic diseases. Therapy of diabetes with insulin is surely the most prominent example, but treatment of anemia with erythropoietin and derivatives or the use of interferons for hepatitis C therapy are examples of similar importance (L. R. Brown, Exp. Opin. Drug Del., 2 (1), 29-42, 2005). Recently, the development of high-yield and robust manufacturing processes for proteins has created an increasing amount of therapeutic proteins including antibodies and fragments thereof and it can be expected that their number will still rise in future. Beside peptide drugs, the class of macromolecular therapeutics includes also other substances, *e.g.* heparins and therapeutic oligonucleotides.

The development of formulations for oral administration of macromolecules is rendered difficult for several reasons. Many macromolecules are unstable under the harsh conditions of the GIT and are degraded pre-systemically in the stomach or small intestine (G. Fricker et al., J. Pept. Sci., 2 (4), 195-211, 1996). Most macromolecular drugs can be classified in the biopharmaceutical classification system as class III, having a high solubility and a low permeation across the intestinal mucosa. According to Lipinski's rule of five, the low permeation is caused by their size (over 500 Da) and their hydrophilicity (usually more than 5 H-bond donors and more than 10 H-bond acceptors) (C. Lipinski et al., Adv. Drug Deliver. Rev., 23 (1-3), 3-25, 1997). In conclusion, even if a certain fraction of the applied macromolecule is not degraded during the GIT passage and reaches the intestinal wall, it is hardly taken up. This usually results in a bioavailability of less than 1% and only a few therapeutically relevant peptide drugs are available as oral dosage forms, e.g. cyclosporine A (microemulsion) or desmopressin (tablet) (E. Ziv et al., Microsc. Res. Tech., 49 (4), 346-52 2000). However, both peptides have a molecular weight below 1500 Da and there is still need for the development of highly innovative drug forms for the delivery of bigger proteins like insulin or human growth hormone (hGH) or of peptidic and non-peptidic drugs with a MW < 2000 Da that are poorly water-soluble, e.g. drugs that are classified as Class II or Class IV drugs according to the Biopharmaceutics Classification System (BCS) developed by the US Food and Drug Administration (FDA).

In general, efforts to increase oral bioavailability of macromolecules, poorly water soluble compounds and poorly permeable compounds focus on either stability improvement in the GIT or enhancement of permeation across the intestinal mucosa or both. Several strategies have been evaluated to overcome physicochemical and metabolic barriers of the GIT including chemical modifications, targeting of intestinal membrane transporters and endocytosis mechanisms, enzyme inhibitors, permeation enhancers, bioadhesive systems and particulate delivery systems, such as polymeric micro- and nanoparticles and liposomes (J. Hamman et al., BioDrugs, 19 (3), 165-177, 2006).

Spontaneous formation of vesicles after hydration of phospholipids, called liposomes, was firstly described in 1965 (A. D. Bangham et al., J. Mol. Biol., 13 (1), 238-52, (1965). Liposomes are of spherical shape and consist of one or several lipid bilayers surrounding an aqueous space. Drug substances can be encapsulated depending on their hydrophilicity either in the aqueous core or in the lipid membrane (A. Jesorka et al., Annu. Rev. Anal. Chem., 1, 801-832, 2008). Soon after their discovery, liposomes were investigated for drug delivery mainly because of their versatility in composition and preparation techniques and their good biocompatibility (F. Szoka et al., Annu. Rev. Biophys. Bioeng., 9, 467-508, 1980). Eventually, efforts in research resulted in the mid 1990's in the approval of several liposomal formulations for parenteral use, mainly in cancer therapy (T. Lian et al., J. Pharm. Sci., 90 (6), 667-680, 2001). However, first approaches to use liposomes for oral peptide delivery where not very encouraging mostly due to a poor reproducibility of the results.

Several methods for liposome preparation have been developed in the last 45 years. A very common approach in the lab scale is the so called film method followed by extrusion or sonication to reduce vesicle size. After hydration of a dried lipid film, multilamellar lipid vesicles (MLV) form spontaneously, but vesicle size is rather large and heterogeneous. During extrusion, MLVs are passed through a filter of defined pore size under mild pressure (B. Mui et al., Meth. Enzymol., 367, 3-14, 2003). This procedure is repeated several times until vesicle size cannot be reduced any further and size distribution has the desired width. This method is very simple, fast and leads to small or large unilamellar vesicles with good size homogeneity.

Vesicular phospholipid gels (VPGs) are described in U.S. Pat. No. 6,399,094 B1 to M. Brandl et al. They are semisolid, aqueous phospholipid dispersions, where liposomes are so tightly packed, that they increase viscosity of the dispersion. Commonly, VPGs were prepared by high pressure homogenization, but recently a new method has been introduced in European Patent Application EP 1674081 A1 by U. Massing based on the principle of dual asymmetric centrifugation (DAC), which is suitable also for the preparation of small amounts of VPGs in lab scale. Advantages of VPGs are their high encapsulation efficiency for hydrophilic drugs and their good storage stability. In U.S. Pat. Appl. No. 2010/0239654 A1 it is disclosed that the DAC method is also suitable for the incorporation of macromolecules, such as protein drugs. It is mentioned that a hydrophilic polymer can be added to the formulation to stabilize the encapsulated protein. The polymer is mixed together with the phospholipids and not with the aqueous phase. Moreover, it is intended to be between the hydrophobic lipid molecules and not in the interior or exterior aqueous space of the liposomes, where it could solidify the liposomal formulation. The formulations described in U.S. Pat. Appl. No. 2010/0239654 A1 have to be liquid or semisolid to assure sufficient syringeability and are not stabilized against leakage or degradation in the GIT. Thus, they are not suited for a direct oral administration with intestinal absorption. In the above patent application, administration in the oral cavity (e.g. buccal) is claimed, but not a peroral application with following absorption of the active substance in the intestine. For oral delivery, solid dosage forms are preferred over liquid or semi-solid dosage forms, because of their better stability, higher dosing accuracy and more convenient way of application. Moreover, U.S. Pat. Appl. No. 2010/0239654 A1 does not specify the polymer and water content of the described pharmaceutical composition.

In European Patent 0393049 B1 by J. Hauton production of liposomes containing a gelling agent in the inner lumen is described. Liposomes are prepared by a common method and subsequently the formulation is diluted to reduce the concentration of the gelling agent in the outer phase below its gelling concentration resulting in a liquid liposome dispersion. These liposomes are not formed as VPGs leading to comparable lower encapsulation efficiency. Furthermore, the concentration of the gelling agent is limited to concentrations in the range between 5% and 10% restricting the stabilizing effect of the gelling agent. The liposomes described in Patent 0393049 are not embedded in a solid matrix, therefore they have to be transferred in an additional production step, such as freeze-drying, to a solid form.

Qun Zeng describes in US 2011/020428 A1 liposomes that contain an internal and an external thermo-reversible hydrogel. The liposomes are prepared by a two-step emulsion method including the use of water-immiscible solvents. These mostly belong to the group of Class 2 Solvents according to the USP and their use should be limited in drug products, because of their toxicity. The external hydrogel is added after formation of the liposomes and the solvents are removed at a temperature below the sol-gel-transition of this hydrogel limiting either the efficiency of the solvent removal or the gel strength of the hydrogel. The suitable hydrogel concentration in the external phase is below 5% (w/v) to assure the formation of a hydrogel, but not a solid matrix.

In U.S. Patent 4,839,111 solid core liposomes are described. They are prepared in a three step solvent-based method that cannot be used for the encapsulation of instable substances like protein drugs. For analytical purposes, the solid core liposomes were fixed with glutaraldehyde and embedded in agarose. During this procedure instable substances like proteins will be most likely denatured and the liposomal dispersion is diluted. Moreover, with the described method it is not possible to produce liposomes smaller than 500 nm, which is required to obtain a sufficient uptake of the nanoparticles through biological membranes. Therefore they are not suitable for oral peptide/protein delivery.

In the Patent WO 2006/103657 A2 by A. Pinhasi and M. Gomberg a solid delivery system for insulin containing a phospholipid and a hydrophilic polymer matrix is described. The phospholipids are not hydrated in the delivery system and form upon contact with the oral cavity liquid micelles, emulsions, liposomes or a mixture thereof. Thus, encapsulation of the drug and the gelling agent into the phospholipid particles and their size cannot be controlled. Furthermore, liposomes are not directly available as such for drug delivery, but have to form over time. Considering these aspects, the described system is not suitable for the delivery of drugs incorporated in liposomes intended for oral use and enteral absorption.

The Patent WO 2004/009053 A2 by M. Farber and J. Farber defines a transmucosal delivery system, where an active agent associated with membrane vesicles, such as liposomes, is evenly dispersed in an external matrix. In the US Patent Application No. 2008/0279921 A1 by V. Albrecht and D. Scheglmann a formulation for the delivery of hydrophobic drugs is delineated. In one embodiment the drug is associated with liposomes that are incorporated in a gel matrix. For both systems, a stabilization of the vesicles by increasing the viscosity of their inner lumen by a gelling agent is not achieved. The delivery systems are manufactured in a two-step process, where the membrane vesicles are formed first and are then mixed with the matrix former, which increases production time and costs.

Up to date, the inventors are not aware of any formulation of liposomes for oral administration other than in the form of aqueous dispersions, or lyophilisates. However, aqueous dispersions of liposomes are not stable and can promote the degradation of the agents contained in the liposomes. On the other hand, the presence of water is mandatory to keep the lipids of the liposomal membrane and encapsulated hydrophilic substances fully hydrated. In the hydrated form, liposomes and the encapsulated substances are more readily available, because they do not need to be re-hydrated after dispersion in the body fluids as it is the case for freeze- or spray-dried formulations. Moreover, lyophilization of liposomes is a cost- and energy-intensive process (E. C. van Winden, Meth. Enzymol., 367, 99-110, 2003). Finally, a control of the release of the agent is not possible when using these dosage forms.

Accordingly, the technical problem underlying the present invention is to provide a composition of liposome-based drugs which can be administered orally in a single-dosed manner (monolithic formulation), provides a good stability of the composition and the agent contained therein, can be produced easily and cost-efficiently in a gentle way to protect the encapsulated agents, can be administered easily and conveniently, does contain the encapsulated agents in their biologically active form, and allows the control of the release of the agent after administration.

The solution to the above technical problem is achieved by the embodiments characterized in the claims.

In particular, in a first aspect, the present invention relates to a solid dosage form comprising:

> liposomes;
> water in an amount of between 5% and 95% (w/w), based on the total mass of the solid dosage form; and
> one or more solidifier(s);
> wherein said solidifier(s)
> form(s) a solid matrix in which said liposomes are embedded, and
> is/are contained in the inner lumen of said liposomes.

For sake of clarification, the liposomes and the one or more solidifier(s) form a system in which the one or more solidifier(s) form(s) a solid matrix in which said liposomes are embedded, and is/are contained in the inner lumen of said liposomes.

The term "liposome" as used herein refers to artificially prepared vesicles composed of lipid bilayers. Liposomes can be used for delivery of agents due to their unique property of encapsulating a region of aqueous solution inside a hydrophobic membrane. Dissolved hydrophilic solutes cannot readily pass through the lipid bilayer. Hydrophobic compounds can be dissolved in the lipid bilayer, and in this way liposomes can carry both hydrophobic and hydrophilic compounds. To deliver the molecules to sites of action, the lipid bilayer can fuse with other bilayers such as cell membranes, thus delivering the liposome contents. By making liposomes in a solution of an agent, it can be delivered to the inner lumen of the liposome. There are three types of liposomes - MLV (multilamellar vesicles) SUV (Small Unilamellar Vesicles) and LUV (Large Unilamellar Vesicles). These are used to deliver different types of drugs. The term "liposomal composition" refers to an emulsion comprising an aqueous solvent in which liposomes are emulsified. The inner lumen of such liposomes is usually filled with the same liquid solvent in which the liposomes are dispersed.

In a preferred embodiment, the liposomes comprised in the solid dosage form of the present invention comprise an agent selected from the group of pharmaceutically active agents and pro-forms thereof, diagnostic agents, nutritional supplements, and cosmetics. However, in a different embodiment, the liposomes comprised in the solid dosage form of the present invention do not comprise any such agent, as the lipids of which the liposomes are composed can already have a desired therapeutical effect by themselves.

The term "dosage form" as used herein refers to the form of an active substance often in combination with auxiliary substances, *i.e.,* excipients, that is directly administrable to a patient. A dosage form is referred to as semi-solid, when it exhibits a yield point at room temperature and is easily deformed by pressure or shear forces, but not by gravity. Semi-solid dosage forms usually contain at least one component that is liquid or semi-solid at room temperature. The active substances are either dissolved or very finely dispersed in the semi-solid dosage form. Typical examples are gels, creams and pastes. Most often, they are used as multiple unit dosage form.

Unlike semi-solid dosage forms, solid dosage forms do not deform substantially at room temperature, even under pressure or shear forces. Typically, they are used as single units, *e.g.* tablets, capsules or films that dissolve in the mouth, but also as multiple unit dosage form like powders or granules. Due to their mechanical stability at room temperature they can be further modified, *e.g.* film-coated. Solid dosage forms prepared by compaction of powders or granules, *i.e.,* tablets, are comparatively hard and brittle. Others like soft or hard capsules deform under pressure, but recover their shape after the pressure is released.

In this context, in a preferred embodiment, the solid dosage form of liposomes of the present invention, is further coated with a polymer layer. Respective polymers are not particularly limited and are known in the art. They include for example chitosan, cellulose-based polymers such as ethyl cellulose, and acrylic acid derivatives such as Eudragit E.

According to the present invention, the one or more solidifier(s) can induce or facilitate the formation of a solid matrix in which the liposomes are embedded, *i.e.,* they are surrounded by said solid matrix on all sides. Further, said one or more solidifier(s) are also be contained in the inner lumen of said liposomes, *i.e.,* in the space that is formed by the surrounding lipid bilayer of the liposome. The one or more solidifier(s) are therefore in the surrounding of the liposomes and concomitantly in the liposomes interior, *i.e.,* the liposomal envelope is embedded from both sides.

The liposomes used in the solid dosage form according to the present invention are not particularly limited to specific lipids. In particular, the lipids used for the generation of said liposomes can be any suitable lipids known in the art. These lipids include - but are not restricted to - cholesterol or derivatives thereof, phospholipids, lysophospholipids or tetraetherlipids. Accordingly, in a preferred embodiment, said liposomes comprise one or more lipids, selected from the group consisting of cholesterol and derivatives thereof, phospholipids, lysophospholipids, and tetraetherlipids. Preferably, said liposomes comprise phospholipids, wherein said phospholipids can be synthetic, semi-synthetic or natural phospholipids, and are preferably selected from the group consisting of egg-phosphatidylcholine (E-PC), dipalmitoyl phosphatidylcholine, and soy-phosphatidylcholine. In general, suitable lipids can be selected from the group consisting of phosphatidylcholines, phosphatidylethanolamines, phosphatidylinosites, phosphatidylserines, cephalines, phosphatidylglycerols, and lysophospholipids. In a particular embodiment of the present invention, the liposomes consist of E-PC and cholesterol, preferably in a ratio of 60:40. In a preferred embodiment, the content of lipids in the solid dosage form of liposomes according to the present invention is at least 5% (w/w) based on the total mass of the solid dosage form, more preferably at least 15% (w/w), at least 25%(w/w) or at least 30% (w/w), wherein at least 30% (w/w) are particularly preferred. The liposomes to be used according to the present invention may further comprise any further suitable agents such as *e.g.* enzyme inhibitors, permeation enhancers, or other lipophilic or hydrophilic substances that can be used for the stabilization of liposomes or for altering liposome properties. Such lipophilic or hydrophilic substances are not particularly limited and are known in the art. They include for example vitamin E, fatty acids, waxes, and mono-, di- and triglycerides.

The lipids used for preparation of these liposomes can also be attached to target seeking structures such as peptide sequences, antibodies, receptor ligands and also surfactants.

In a preferred embodiment, the liposomes comprised in the solid dosage form of the present invention exhibit a Z-Average measured by dynamic light scattering after dilution in aqueous medium of at most 1000 nm and a polydispersity index of at most 0.7, more preferably a Z-Average of at most 750 nm and a polydispersity index of at most 0.5, a Z-Average of at most 450 nm and a polydispersity index of at most 0.4, or a Z-Average of at most 350 nm and a polydispersity index of at most 0.3, where a Z-Average of at most 350 nm and a polydispersity index of at most 0.3 is particularly preferred.

In a preferred embodiment, the liposomes comprised in the solid dosage form of the present invention are densely packed with only little exterior aqueous phase between the single vesicles. These types of liposome dispersions are also referred to as vesicular phospholipid gels (VPGs). VPGs as such are semisolid, since the liposomes therein are so tightly packed that they increase the viscosity of the dispersion. VPGs can be diluted with water or aqueous buffer and form conventional, liquid liposome dispersions.

Methods for the generation of liposomes and for the generation of densely packed liposome dispersions are not particularly limited and are known in the art. They include for example high pressure homogenization and dual asymmetric centrifugation (DAC). Details concerning these methods are given below.

As used herein, the solid dosage form according to the present invention may be referred to as "solidified liposomes", "matrix liposomes", "jellied liposomes" or "jellied VPGs".

The solidifier(s) to be used in the solid dosage form according to the present invention is/are not particularly limited and include(s) any suitable solidifier(s) known in the art that can be solidified after preparation of the solid dosage form. Further, the solidifier(s) can be any type of solidifier(s) whose viscosity is not only dependent on its concentration, but also on temperature, ionic strength, pH or other parameters. The solidifier(s) to be used in the solid dosage form of the present invention have/has to dissolve, to swell or be degradable in or by physiological body fluids or other aqueous solutions. Preferably, said solidifier(s) is/are selected from the group consisting of polymers that are also used for the preparation of pharmaceutical capsules, *i.e.*, hydroxypropyl methylcellulose, pullulan, hydroxypropyl starch and gelatine, wherein gelatine is particularly preferred.

Gelatine is a water-soluble biopolymer with a molecular weight predominantly between 20 to 250 KD derived from collagenous proteins. It can be sourced from different animals, like pig, cow, chicken or fish. Typically, gelatine is isolated from either skin and rind with acid extraction or from hide and bone with an alkaline pretreatment, followed by acid extraction. The different ways of extractions are however not exclusive for one type of tissue. Both types differ in their isoelectric point: alkaline treated gelatine has usually an isoelectric point around pH 5.0 and acid treated between pH 8.0 and 9.0. Characteristic for gelatine is its amino acid composition with a high amount of glycine (approx. 27%), proline, hydroxyproline and the acidic amino acids aspartic and glutamic acid (approx. 15%) and the basic lysine and arginine (approx. 18.5%). Gelatine is soluble in hot water and sets to a gel upon cooling. This process is reversible and can be repeated several times with only little change in the gelling properties of the gelatine. The mechanical properties of the gel are influenced among others by the physico-chemical properties of the gelatine, its concentration and the temperature. Highly concentrated gelatine solutions can be used to form solid sheets or gelatine hard capsules after cooling. By addition of plasticizers, *e.g.* glycerol, more flexible structures with high tensile strength can be formed. These mixtures are used to produce gelatine soft capsules.

In another preferred embodiment, said solidifier(s) is/are selected from the group consisting of curable polymers, alginate, cellulose acetate phthalate, sodium carboxymethylcellulose, hydroxy ethylcellulose, hydroxy propylcellulose, methylcellulose, methylhydroxy ethylcellulose, polyacrylic acid and derivatives thereof, pectin, polyvinyl pyrrolidone (PVP), agarose, alginic acid, collagen, proteins, xanthan gum, carrageenan, tragacanth, chitosan, acacia, polyethylene glycol (PEG) having preferably a molecular weight from 1200 to 6000 Da, hydroxypropyl methylcellulose, pullulan, hydroxypropyl starch and gelatine.

The content of the solidifier in the solid dosage form is dependent on the type of solidifier used, wherein suitable content ranges are known in the art. In preferred embodiments, when the solidifier is alginate, the content thereof in the solid dosage form is 2 to 8% (w/w) based on the total mass of the solid dosage form; when the solidifier is agarose, the content thereof in the solid dosage form is 0.5 to 4% (w/w) based on the total mass of the solid dosage form; when the solidifier is lower weight grade PEG, the content thereof in the solid dosage form is 50 to 80% (w/w) based on the total mass of the solid dosage form; and when the solidifier is higher weight grade PEG, the content thereof in the solid dosage form is 20 to 50% (w/w) based on the total mass of the solid dosage form. For other solidifiers, and in particular in case the solidifier is gelatine, the content of the solidifier in the solid dosage form is preferably 1.5 to 25% (w/w) based on the total mass of the solid dosage form, more preferably 3 to 20% (w/w), more preferably 5 to 15% (w/w). In further preferred embodiments, the content of the solidifier in the solid dosage form is between 0% and 50%, based on the total mass of the solid dosage form, more preferably between 1% and 30%, between 2% and 25% or between 5% and 20%, where between 5% and 20% is particularly preferred.

In a preferred embodiment of the solid dosage form according to the present invention the lipid is a phospholipid and/or in addition a tetraether lipid.

The content of water in the solid dosage form is dependent on the type of lipids, solidifier and agent used. According to the present invention, the content of water in the solid dosage form is between 5% and 95% (w/w), based on the total mass of the solid dosage form, more preferably between 20% and 75% (w/w), between 35% and 65% (w/w) or between 45% and 55% (w/w), where between 45% and 55% (w/w) is particularly preferred. To the water other water-soluble substances like salts, antioxidants, surfactants, sugars or other water-soluble excipients can be added to stabilize the liposomes or the enclosed agent or to modify pH, ionic strength or other physicochemical parameters of the solid dosage form. Furthermore, substances that enhance the bioavailability of enclosed active agents, like enzyme inhibitors, tight junction modulators or chelating agents can be added.

In a preferred embodiment, the agent comprised in the liposomes of the present invention is a pharmaceutically active agent, a pro-form thereof, a diagnostic or a nutritional supplement. Said pharmaceutically active agent is not particularly limited and includes any agents the administration of which as a liposomal drug is of interest. Accordingly, pharmaceutically active agents can be selected from the group consisting of protein drugs, peptide drugs, nucleic acid drugs, and small molecule drugs. In particular, the pharmaceutically active agent can be selected from the group consisting of human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, or toxins, virus-derived vaccine antigens, octreotide, cyclosporine, rifampycin, lopinavir, ritonavir, vancomycin, telavancin, oritavancin, dalbavancin, bisphosphonates, itraconazole, danazol, paclitaxel, cyclosporin, naproxen, capsaicin, albuterol sulfate, terbutaline sulfate, diphenhydramine hydrochloride, chlorpheniramine maleate, loratidine hydrochloride, fexofenadine hydrochloride, phenylbutazone, nifedipine, carbamazepine, naproxen, cyclosporin, betamethasone, danazol, dexamethasone, prednisone, hydrocortisone, 17 beta-estradiol, ketoconazole, mefenamic acid, beclomethasone, alprazolam, midazolam, miconazole, ibuprofen, ketoprofen, prednisolone, methylprednisone, phenytoin, testosterone, flunisolide, diflunisal, budesonide, fluticasone, insulin, acylated insulin, glucagon-like peptide, acylated glucagon-like peptide, exenatide, lixisenatide, dulaglutide, liraglutide, albiglutide, taspoglutide, C-Peptide, erythropoietin, calcitonin, luteinizing hormone, prolactin, adrenocorticotropic hormone, leuprolide, interferon alpha-2b, interferon beta-la, sargramostim, aldesleukin, interferon alpha-2a, interferon alpha-n3alpha-proteinase inhibitor, etidronate, nafarelin, chorionic gonadotropin, prostaglandin E2, epoprostenol, acarbose, metformin, desmopressin, cyclodextrin, antibiotics, antifungal drugs, steroids, anticancer drugs, analgesics, anti-inflammatory agents, anthelmintics, anti-arrhythmic agents, penicillins, anticoagulants, antidepressants, antidiabetic agents, antiepileptics, antihistamines, antihypertensive agents, antimuscarinic agents, antimycobacterial agents, antineoplastic agents, CNS-active agents, immunosuppressants, antithyroid agents, antiviral agents, anxiolytic sedatives, hypnotics, neuroleptics, astringents, beta-adrenoceptor blocking agents, blood products and substitutes, cardiacinotropic agents, contrast media, corticosteroids, cough suppressants, expectorants, mucolytics, diuretics, dopaminergics, antiparkinsonian agents, hemostatics, immunological agents, lipid regulating agents, muscle relaxants, parasympathomimetics, parathyroid calcitonin, prostaglandins, radiopharmaceuticals, sex hormones, steroids, anti-allergic agents, stimulants, anoretics, sympathomimetics, thyroid agents, vasodilators, xanthines, heparins, therapeutic oligonucleotides, somatostatins and analogues thereof, and pharmacologically acceptable organic and inorganic salts or metal complexes thereof.

In a preferred embodiment, the content of pharmaceutically active agent or a pro-form thereof in the solid dosage form according to the present invention is above 0% (w/w) and at most 50% based on the total mass of the solid dosage form, more preferably at most 30% (w/w), at most 20%(w/w) or at most 10% (w/w), wherein at most 10% (w/w) are particularly preferred.

In another embodiment, the agent comprised in the liposomes of the present invention is a diagnostic agent. Said diagnostic agent is not particularly limited and includes any agents the administration of which as a liposomal diagnostic is of interest, e.g. for gamma-scintigraphy, magnetic resonance imaging (MRI), computed tomography (CT) imaging, PET-Scan and sonography. For example, the diagnostic agent could be Gd-DTPA, Indocyanine green, DTPA-PE, DTPA-NPLL-NGPE and others.

In a preferred embodiment, the content of diagnostic agent or a pro-form thereof in the solid dosage form according to the present invention is above 0% (w/w) and at most 50% based on the total mass of the solid dosage form, more preferably at most 35% (w/w), at most 25%(w/w) or at most 15% (w/w), wherein at most 15% (w/w) are particularly preferred.

In a further embodiment, the agent comprised in the liposomes of the present invention is a nutritional supplement. Said nutritional supplement is not particularly limited and includes any nutritional supplement the administration of which as a liposomal formulation is of interest. Accordingly, nutritional supplements can be selected from the group consisting of hyaluronic acid, chondroitin, bromelain, papain, whey protein, collagen hydrolysates, lipophilic vitamins such as vitamins D, E, and K, carotenoids, lycopene, omega-3 fatty acids, plant sterols, alpha lipoic acid, coenzyme Q10, curcumin, and iron.

In a preferred embodiment, the content of nutritional supplement or a pro-form thereof in the solid dosage form according to the present invention is above 0% (w/w) and at most 50% based on the total mass of the solid dosage form, more preferably at most 35% (w/w), at most 25%(w/w) or at most 15% (w/w), wherein at most 15% (w/w) are particularly preferred.

In a further embodiment, the agent comprised in the liposomes of the present invention is a cosmetic agent such as an antioxidant, antimicrobial, antiseborrheic, bleaching, or coloring agent.

In a preferred embodiment, the content of cosmetic agent or a pro-form thereof in the solid dosage form according to the present invention is above 0% (w/w) and at most 50% based on the total mass of the solid dosage form more preferably at most 35% (w/w), at most 25%(w/w) or at most 15% (w/w), wherein at most 15% (w/w) are particularly preferred.

According to the present invention, the solid dosage form is preferably for oral or topical or parenteral administration. Other routes of application are also feasible (*e.g.* parenteral, buccal, topical).

In a second aspect, the present invention relates to a method for the production of the solid dosage form of the present invention, said method comprising the step of:

(a) preparing liposomes in a dispersion comprising (i) water in an amount of between 5% and 95% (w/w), based on the total mass of the dispersion, and (ii) one or more solidifier(s);
(b) forming the composition obtained in step (a) to a desired dosage form; and
(c) letting said composition solidify.

In a preferred embodiment, the liposomes comprised in the solid dosage form produced by the method comprise an agent selected from the group of pharmaceutically active agents and pro-forms thereof, diagnostic agents, nutritional supplements, and cosmetics. Accordingly, in this preferred embodiment, the dispersion used in step (a) of the method of the present invention comprises (iii) an agent selected from the group of pharmaceutically active agents and pro-forms thereof, diagnostic agents, nutritional supplements, and cosmetics.

In this aspect, all relevant definitions and embodiments described for the first aspect of the present invention apply in an analogous manner to the second aspect of the present invention. In particular, the liposomes, solidifier(s), and agent, as well as the respective contents thereof are as defined above.

Methods for preparing liposomes or VPGs are not particularly limited and are known in the art. Preferably, liposomes or VPGs are formed by high pressure homogenization or by dual asymmetric centrifugation (DAC). Briefly, in the high pressure homogenization method, lipids are dissolved, mixed, and the solution dried to remove any solvent traces and the resulting lipid films are hydrated in buffer or the lipids are directly dispersed in buffer. The resulting multilamellar vesicles (MLVs) are reduced in size in a fluid jet or piston gap high pressure homogenizer in the above DAC method, lipid films are generated in a similar manner in suitable reaction vessels and subsequently processed in a speed mixer as known in the art.

In a preferred embodiment, the liposomes are prepared by a method, selected from the group consisting of high pressure homogenization and dual asymmetric centrifugation (DAC). In a further preferred embodiment, the liposomes are prepared as vesicular phospholipid gel (VPG). In another preferred embodiment, the content of lipids in the solid dosage form is at least 25% (w/w) based on the total mass of the solid dosage form.

According to the method of the present invention, liposomes are preferably prepared in a dispersion comprising the solidifier(s) and, if present, the agent, *i.e.,* the solidifier(s) and, if present, the agent are contained in the buffer that is added to the lipid films prior to high pressure homogenization or speed mixing. In this manner, the solid dosage form of the present invention can be obtained in a single step, with the solidifier(s) being contained in the inner lumen of the resulting liposomes, as well as forming a solid matrix in which said liposomes are embedded after solidification of said solidifier(s). The content of agent in the above dispersion, if present, is chosen such that a desired content of said agent in the final solid dosage form is achieved. The content of the solidifier(s) in the above dispersion is chosen such that the desired content of the solidifier(s) in the solid dosage from is achieved, wherein suitable content ranges in the above dispersion can be easily determined by the person skilled in the art. Preferably, in case the solidifier is gelatine, the dispersion used in step (a) of the method of the present invention comprises said gelatine in an amount of 1.5 to 25% (w/w) based on the total mass of the dispersion. The content of the solidifier(s) in the solid dosage form is dependent on the type of solidifier(s) used, wherein suitable content ranges are known in the art. In preferred embodiments, when the solidifier is alginate, the content thereof in the solid dosage form is 2 to 8% (w/w) based on the total mass of the solid dosage form; when the solidifier is agarose, the content thereof in the solid dosage form is 0.5 to 4% (w/w) based on the total mass of the solid dosage form; when the solidifier is lower weight grade PEG, the content thereof in the solid dosage form is 50 to 80% (w/w) based on the total mass of the solid dosage form; and when the solidifier is higher weight grade PEG, the content thereof in the solid dosage form is 20 to 50% (w/w) based on the total mass of the solid dosage form. For other solidifiers, and in particular in case the solidifier is gelatine, the content of the solidifier in the solid dosage form is 1.5 to 25% (w/w) based on the total mass of the solid dosage form, more preferably 3 to 20% (w/w), more preferably 5 to 15% (w/w). In further preferred embodiments, the content of the solidifier(s) in the solid dosage form is between 0% and 50%, based on the total mass of the solid dosage form, more preferably between 1% and 30%, between 2% and 25% or between 5% and 20%, where between 5% and 20% is particularly preferred.

Means of forming the composition obtained in step (a) of the method of the present invention to a desired dosage form, which composition is a semi-solid composition prior to solidification in step (c), are not particularly limited and are known in the art. They include any suitable means of shaping a semi-solid composition. This could be done for example by transferring the semi-solid composition after preparation in molds that have the desired form of the final solid dosage form, e.g. an oval, capsule like shape, a lentil form or a flat sheet. Or by using a calendaring system that molds the semi-solid form in a continuous way in the desired form. Moreover, the composition could be given a round shape by dropping or spraying the composition into a stream of cold air or a cooling bath. Or by dropping or spraying the composition into a stream or a solution of substances that initiate the solidification of the composition as described below. Instead of giving the composition the final shape before solidification, it could be solidified in larger sheets or cylinders or other forms and cut into the final shape by use of a knife or a laser cutting system.

Further, means for letting said composition solidify are not particularly limited and are known in the art. In case the solidifier shows are temperature dependent sol-gel transition, e.g. gelatine, they include for example any means of storing said composition at a temperature below the melting temperature of the composition for a sufficient amount of time to let the composition solidify. The cooling could be for example done by storing the shaped composition in a fridge or cooling room, by letting a cold air stream run over the composition, by actively cooling the molds that are used for shaping the composition or by letting the semi-solid composition drip into a cooling bath or cold air stream. In case the solidifier is a polymer that exhibits a sol-gel transition in dependence of ion strength, pH or other physicochemical parameters, these parameters could be changed by addition of suitable substances, e.g. salts, acids or bases to the semi-solid composition. In case the solidifier is crosslinkable, e.g. alginate, they include for example the addition of Ca2+ ions or other substances that start the crosslinking reaction. In case of substances that could be polymerized after preparation of the semi-solid composition the solidification could be started by addition of a radical starter or by UV-light or other radiation. The above mentioned substances could be added by mixing them with the composition preferably by DAC or high-pressure homogenization just shortly before shaping the composition in the desired final form or the semi-solid composition could be dropped in a solution or a stream of said substances. Furthermore, above mentioned substances could be co-sprayed with the semi-solid composition to mix them and to form small droplets of the solidified composition at the same time. It is also possible to initiate the solidification process of the composition by reducing the water content and increasing the concentration of the solidifier, e.g. by freeze-, spray- or tray-drying.

In a preferred embodiment of the method according to the present invention, the solidifier is gelatine and the dispersion used in step (a) comprises said gelatine in an amount of 1.5 to 25% (w/w) based on the total mass of the dispersion.

In another preferred embodiment, the method of the present invention further comprises the step of coating the solidified solid dosage form with a polymer layer after step (c). In this embodiment, the polymer is preferably as defined above. Methods for coating a solid dosage form with a suitable polymer layer are not particularly limited and are known in the art.

The present invention is based on the finding that by way of adding a solidifier which can be solidified during the preparation of liposomes, and subsequent forming and solidifying of a solid dosage form, solid forms of liposome-based drugs can be obtained which are suitable for single-dosed oral administration. In these compositions, liposomes are present in a stabilized form within a solid matrix formed by the solidifier. After oral administration of the composition, said matrix dissolves in the gastrointestinal fluid and releases the liposomes. The solidifier in the inner lumen of the liposomes remains and increases the viscosity of the liquid core substantially. The degree of viscosity can be modified by appropriate preparation protocols. Thus, leakage of substances from inside the liposomes to the surrounding medium and vice versa is reduced. In addition, the structural integrity of the lipid bilayer is increased by the scaffold formed by the solidifier inside the liposome. By way of controlling the content of solidifier in the composition, the release kinetics of the liposomes can be regulated. Further, the solid dosage form can be coated with a polymer layer, e.g. a functionalized polymer layer.

According to the method of the present invention, surprisingly a solid dosage form can be produced wherein liposomes are embedded in a solid matrix in a high density. Production steps such as lyophilization or the use of high amounts of matrix-forming substances are not necessary. In this context, high density liposomes such as VPGs have so far only been available in a semi-solid condition. It is not possible to form solid liposomal formulations by reducing the water content during preparation, since in this case no liposomes would be formed.

The solid dosage forms of liposomes of the present invention provide a fast availability of the liposomes after administration. Despite the fact that the composition of the present invention forms a solid after preparation it contains a surprisingly high amount of water.

Commonly solid dosage forms like tablets or powders contain only a residual amount of water below 1.5%. Even solid dosage forms that contain polymers, *e.g.* soft or hard capsules or solid dispersions have usually a water content below 5% based on the total mass of the dosage form. On the other hand, in order to be biologically active many agents, especially peptides and proteins need to be fully hydrated and surrounded by aqueous medium. Also the amphiphiles in liposomal membranes need to be hydrated to form a stable bilayer or, in case of tetraether lipids a stable monolayer. Further, said dosage forms do not necessitate any preparatory steps before oral or topical administration such as a reconstitution. Moreover, said dosage forms can be easily and conveniently administered orally in a single-dosed manner, provide a superior storage stability, as well as an improved dosage accuracy, and can be produced in an easy and cost-efficient manner. Furthermore, said dosage forms allow the control of the release kinetics of the pharmaceutically active agent after administration. Finally and importantly, the increase of viscosity of the inner aqueous part of the liposomes of the present invention increases their stability in the gastro-intestinal tract.

The figures show:

Figure 1:
Influence of gelatine on size (upper panel) and polydispersity (lower panel) of liposomes.

Figure 2:
Size (upper panel) and polydispersity (lower panel) of liposomes before and after jellification and redispersion in buffer.

Figure 3:
Release of liposomes over time of solidified VPGs containing different concentrations of gelatine dissolved in simulated intestinal fluid.

Figure 4:
Size (upper panel) and polydispersity (lower panel) of solidified matrix liposomes containing different concentrations of gelatine over time after dissolving them in simulated intestinal fluid.

Figure 5:
Differences in derived count rate overtime between samples of 20% gelatine matrix liposome gels solidified in different masses and dissolved in simulated intestinal fluid.

Figure 6:
Encapsulation efficiency of matrix liposomes containing different amounts of gelatine as determined by comparing detected emission of liposome fractions with un-columned liposomes.

Figure 7:
Comparison of the encapsulation efficiency of liposomes prepared in a direct manner and solidified liposomes.

Figure 8:
Stability of liposomes containing 10% gelatine vs. conventional liposomes under acidic conditions (upper panel) and in the presence of bile salts (lower panel) as determined by carboxyfluorescein release.

Figure 9:
Transmission electron microscopy image of liposomes containing gelatine and human growth hormone (hGH).

Figure 10:
Size (Z-Average) and size distribution (PDI) of matrix liposomes with 10% and 20% gelatine directly after preparation and after three years of storage at 4 °C.

The present invention will now be further illustrated in the following examples without being limited thereto.

**Examples**

Example 1:

Liposome preparation and analysis of size and dispersity

*Material and methods:*

Egg-phosphatidylcholine (E-PC) was obtained from Lipoid GmbH (Ludwigshafen, Germany), cholesterol from Sigma-Aldrich (Taufkirchen, Germany) and lime-bone (LB) gelatine from Gelita AG (Eberbach, Germany). All other chemicals were obtained in the highest purity from the usual commercial sources.

E-PC and cholesterol were dissolved in chloroform/methanol 9:1 and then mixed to achieve an E-PC to cholesterol ratio of 60 to 40. Subsequently, the solvent was evaporated using a Rotavapor-R (Büchi Labortechnik AG, Flawil, Switzerland). The dried lipid mixture was weighed into a 2 ml Eppendorf cup and phosphate buffered saline (PBS) (pH 7.4, NaCl 135 mM, KCl 3 mM, $Na_2HPO_4$ 8 mM, $KH_2PO_4$ 1,5 mM) or gelatine (5% to 25% (w/v) in bidistilled water) in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were either further diluted with PBS in a 30 s mixing step directly after speed mixing to obtain liposomes with the desired final lipid concentration or the VPGs were allowed to solidify. Therefore, cups were stored after mixing at 45 °C to avoid immediate solidification. The gel was centrifuged for 1 min at 13.2 rpm (Centrifuge 5415D, Eppendorf, Hamburg, Germany) through a polyamide monofil filter (80μm, neoLab Migge Laborbedarf-Vertriebs GmbH, Heidelberg, Germany) to separate the gelatine mass from the beads used for speed mixing. Cups were stored refrigerated over night to allow solidification of the gelatine/liposome gels. Next day, they were diluted in buffer to the desired lipid concentration at 37 °C.

Liposomes were diluted with PBS to an appropriate concentration and Z-average and poly-dispersity index (PDI) was determined using a Zetasizer® 3000 HS (Malvern, Works, UK) in the automatic mode.

*Results:*

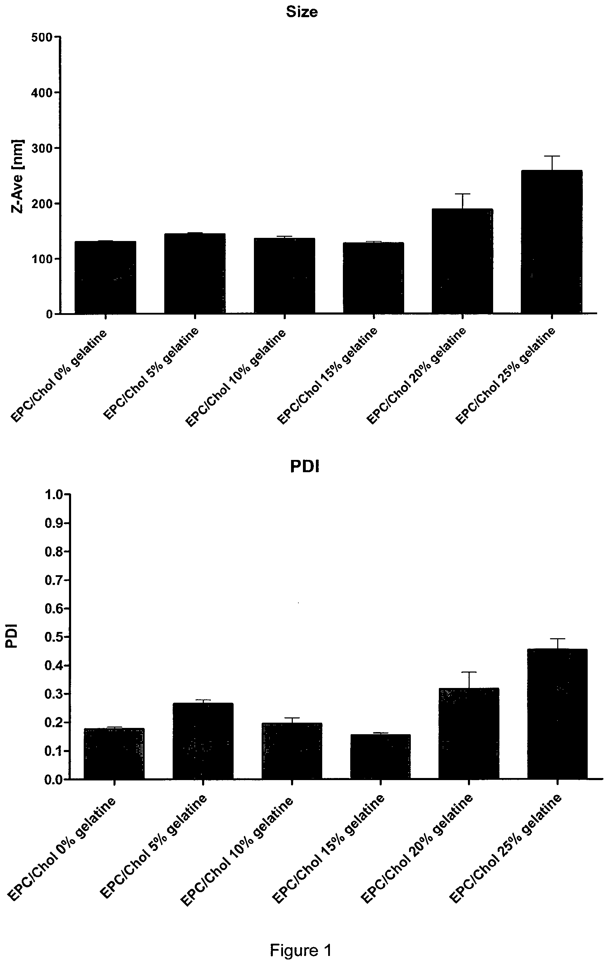

Fig. 1 shows the influence of gelatine on size and polydispersity of liposomes. Surprisingly, the addition of a gelling agent does not influence liposome appearance when used in a concentration up to 15%. U. Massing et al. found in their study that liposome quality with respect to size and polydispersity can decrease with increasing viscosity (Massing et al. Dual asymmetric centrifugation (DAC)-A new technique for liposome preparation. J Control Release (2008) vol. 125 (1) pp. 16-24). With an increasing amount of gelatine (higher than 20%), quality of liposome dispersions decreases slightly compared to normal liposome dispersions in buffer. It was possible to prepare gelatine liposome dispersions in the same quality as usual liposome dispersions in buffer for most of the investigated concentrations of gelatine. The prepared liposome dispersions are intended for the use as oral dosage form and therefore the achieved quality of the gelatine liposome dispersions can be considered as sufficient.

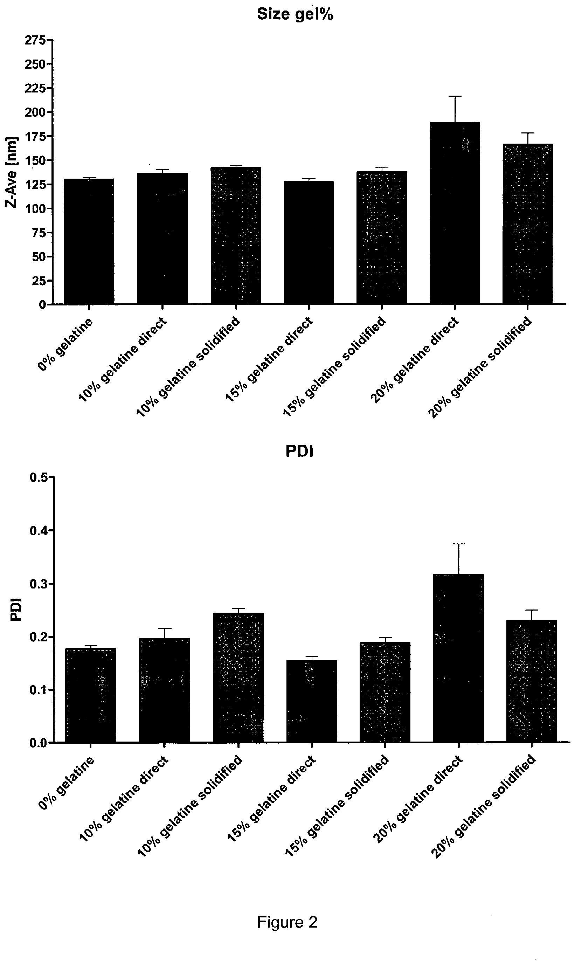

Fig. 2 shows size and dispersity of liposomes before and after jellification and redispersion in buffer. Liposomes prepared normally (direct) compared to solidified liposomes showed not significant differences in size and dispersity.

Example 2:

Dissolution of matrix liposomes

*Material and methods:*

Egg-phosphatidylcholine (E-PC) was obtained from Lipoid GmbH (Ludwigshafen, Germany), cholesterol from Sigma-Aldrich (Taufkirchen, Germany) and lime-bone (LB) gelatine from Gelita AG (Eberbach, Germany). All other chemicals were obtained in the highest purity from the usual commercial sources.

E-PC and cholesterol were dissolved in chloroform/methanol 9:1 and then mixed to achieve an E-PC to cholesterol ratio of 60 to 40. Subsequently, the solvent was evaporated using a Rotavapor-R (Büchi Labortechnik AG, Flawil, Switzerland). The dried lipid mixture was weighed into a 2 ml Eppendorf cup and gelatine solution (10%, 15% and 20% (w/v) in bidistilled water) in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were either further diluted with PBS in a 30 s mixing step directly after speed mixing to obtain liposomes with the desired final lipid concentration or the VPGs were allowed to solidify. Therefore, cups were stored after mixing at 45 °C to avoid immediate solidification. The gel was centrifuged for 1 min at 13.2 rpm (Centrifuge 5415D, Eppendorf, Hamburg, Germany) through a polyamide monofil filter (80μm, neoLab Migge Laborbedarf-Vertriebs GmbH, Heidelberg, Germany) to separate the gelatine mass from the beads used for speed mixing. After separation, the gel was shaped in equivalent sized cylinders (5 mm diameter and 10 mm) and jellified in the fridge.

To assess the dissolution behavior of solidified VPGs and their redispersion into liposomes a paddle dissolution apparatus (PHARMA TEST Typ PTW S III Dissolution tester, Hainburg, Germany) with simulated intestinal fluid (SIF) (pH 6.5) at 37 °C and a volume of 900 ml was used. Samples were taken at different time over two hours. In case of un-dissolved solidified liposome gel an additional sample was taken after 180 min. Collected samples were analyzed by PCS as described in Example 1 and Derived Count Rate, size and PDI was determined.

*Results:*

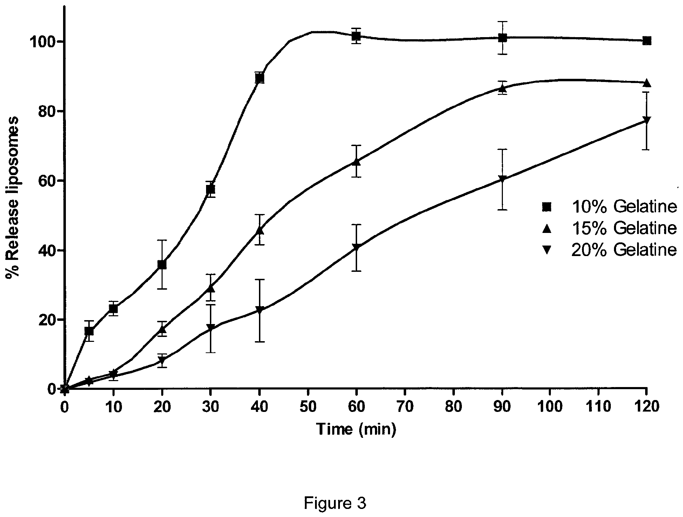

Fig. 3 shows the release of liposomes over time of solidified VPGs containing different concentrations of gelatine dissolved in simulated intestinal fluid (SIF).

All three formulations of solidified matrix liposomes containing different concentrations of gelatine showed an increasing release of liposomes over time after dissolution in SIF, whereby dissolution speed was clearly dependent on gelatine concentration in the formulations.

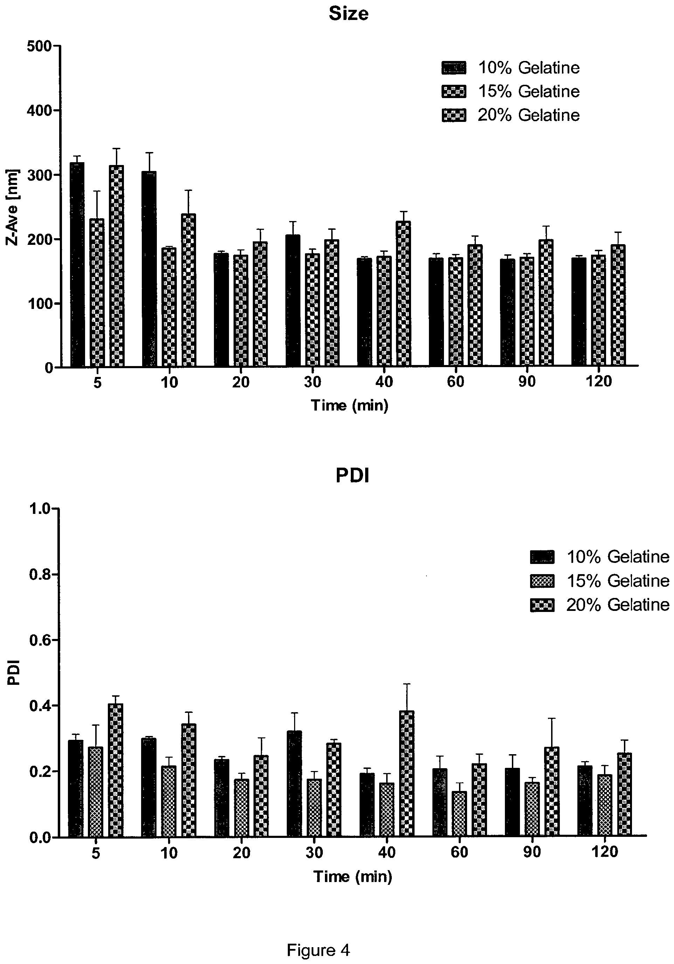

Size and size distribution data show the direct redispersion of the composition to small liposomes with a narrow size distribution (Fig. 4). This is a clear advantage over other conventional solidification methods for liposomes, such as freeze-drying, where the liposomes are de-hydrated during the drying process. Phospholipids in dried-liposomes undergo a phase transition during re-hydration. During phase transition phospholipid bilayers can become leaky especially for smaller hydrophilic drug molecules, which can lead to significantly reduced encapsulation efficiency (Crowe et al. Is Vitrification Sufficient to Preserve Liposomes during Freeze-Drying?. Cryobiology (1994) vol. 31 (4) pp. 355 - 366). Moreover, depending on the concentration of the gelling agent used Matrix Liposomes show a Zero Order release kinetic, which is advantageous, when a constant drug plasma concentration overtime is required.

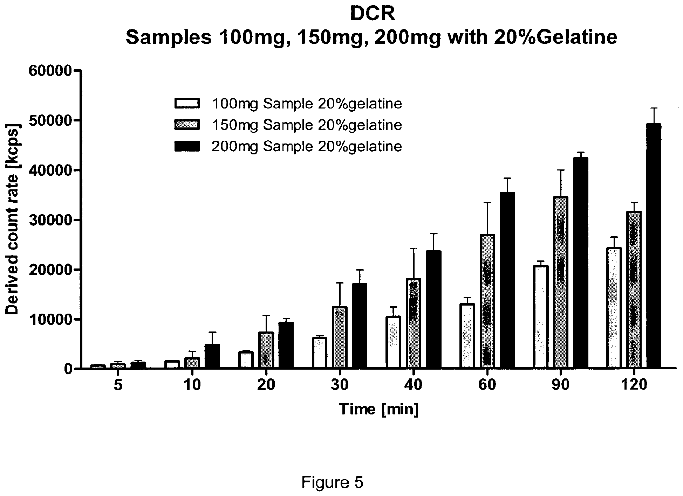

Fig. 5 shows the differences between samples of 20% (w/v) gelatine Matrix Liposome gels solidified in masses of 100, 150 or 200 mg. Samples were dissolved in a dissolution test under the conditions described above. The graph shows the expected result with an increasing derived count rate over time. The higher the weight of the samples, the higher the surface of the dosage form and therefore the higher derived count rate on a selected time point. Over time the different samples dissolve in a similar rate related to the increase of derived count rate.

Example 3:

Encapsulation efficiency of matrix liposomes

*Material and methods:*

Egg-phosphatidylcholine (E-PC) was obtained from Lipoid GmbH (Ludwigshafen, Germany). Cholesterol and fluorescein isothiocyanate-dextran (Mw 70000 Da, FITC-dextran) were purchased from Sigma-Aldrich (Taufkirchen, Germany). Lime-bone (LB) gelatine was obtained from Gelita AG (Eberbach, Germany), Triton-X from Roth GmbH & Co KG (Karlsruhe, Germany) and 5(6)-carboxyfluorescein (CF) from Serva (Heidelberg, Germany). All other chemicals were obtained in the highest purity from the usual commercial sources.

E-PC and cholesterol were dissolved in chloroform/methanol 9:1 and then mixed to achieve an E-PC to cholesterol ratio of 60 to 40. Subsequently, the solvent was evaporated using a Rotavapor-R (Büchi Labortechnik AG, Flawil, Switzerland). The dried lipid mixture was weighed into a 2 ml Eppendorf cup and either CF 50 mM or FITC-dextran (10 mg/ml) in phosphate buffered saline (PBS) (pH 7.4, NaCl 135 mM, KCl 3 mM, $Na_2HPO_4$ 8 mM, $KH_2PO_4$ 1,5 mM) or in gelatine (10%, 15% and 20% (w/v) in bidistilled water) in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were either further diluted with PBS in a 30 s mixing step directly after speed mixing to obtain liposomes with the desired final lipid concentration or the VPGs were allowed to solidify as described above.

For comparison, liposomes were prepared by the conventional extrusion technique. After film formation, the lipid film was hydrated with CF 50 mM to a lipid concentration of 200 mM. The dispersion was then sonicated in a bath type sonicator for 2 h (Elmasonic S 300 H, Elma GmbH & Co. KG, Singen, Germany) and extruded 21 times through a 200-nm membrane using a Liposo-Fast extruder (Avestin, Ludwigshafen, Germany). Freeze drying was performed in a Delta 1-20 KD freeze drier (Christ, Osterode am Harz, Germany) under following conditions:-40°C for 6 h (freezing), -30°C for 40 h (primary drying), 15°C for 8 h (secondary drying). 10% sucrose was used as cryoprotectant. Liposomes were redispersed to the initial concentration prior to determination of the encapsulation efficiency.

Size exclusion chromatography was used to separate free FITC-dextran or CF of encapsulated marker. A Sepharose CL-4B column (GE-Healthcare, Freiburg, Germany) to separate free FITC-dextran of encapsulated marker and a prepacked PD-10 desalting column (GE-Healthcare, Freiburg, Germany) to separate encapsulated CF and free CF was used. Columns were eluted with PBS and liposomes and free marker fraction were collected separately.

Liposome fraction, free marker fraction and un-columned liposome dispersion were analyzed by detecting fluorescence of absorbed light in a Fluoroskan Ascent microplate fluorometer (Thermo Fisher Scientific Inc., Waltham, Massachusetts, USA). Therefore, all samples were diluted in Triton 1 % to destroy liposomes. A black 96-well-plate was used for the measurement. Both markers were detected at an excitation wavelength of 485 and an emission wavelength of 520 nm. Encapsulation efficiency (EE%) was determined according to following equation:

$$EE\% = \frac{FE_{lip}}{FE_{lip} + FE_{free}} \cdot 100\%$$

where $FE_{lip}$ is the fluorescence emission of the liposome fraction and $FE_{free}$ of the free marker fraction after correction of the dilution.

*Results:*

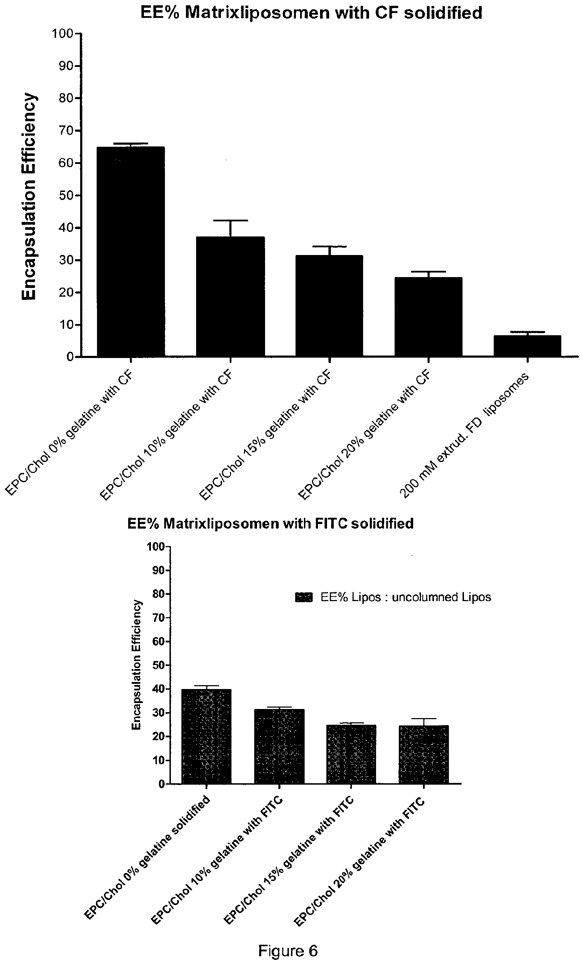

Encapsulation efficiency was calculated by comparing detected emission of liposome fraction with un-columned liposomes. As shown in the size and size distribution experiment, higher concentration of gelatine leads to bigger liposomes and therefore higher encapsulation of compounds might be expected. Increasing concentration of gelatine up to 15% has no pronounced effects on size and size distribution. A decreasing encapsulation efficiency for the small molecular marker CF could be observed with increasing gelatine concentration up to 15% (Fig. 6). In terms of the macromolecular marker, the encapsulation efficiency decreased only slightly and was even at the highest tested gelatine concentration around 30%. However, even with 20% gelatine, the encapsulation efficiency is almost four times higher compared to freeze-dried and extruded liposomes with a lipid concentration of 200 mM (Fig. 6)

Freeze-drying is the preferred method for preparation of solid liposome-based drug forms, although it is very time and energy consuming. The lipid concentration of 200 mM is close to the highest concentration, which is still manufacturable with the common preparation methods. It can be assumed that in commercial products the lipid concentration and thus the encapsulation efficiency would be lower.

Reason for the reduced encapsulation efficiency with increasing gelatine concentration might be the increasing osmotic pressure with higher gelatine concentration. This can lead upon dilution to small membrane defects, which allow the permeation of CF, but not of the larger FITC-dextran.

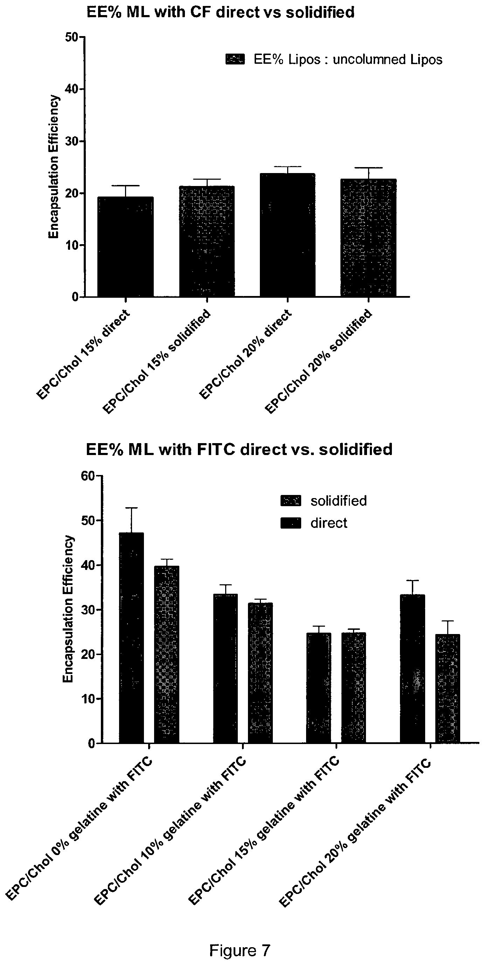

Liposomes were prepared in a direct and a solidified method. If these two methods are compared, in obtaining encapsulation efficiency there is not a significant difference between liposomes prepared directly and solidified liposomes. The jellification has no influence on encapsulation efficiency of either high molecular weight compounds or smaller compounds (Fig. 7)

Example 4:

Stability in simulated gastrointestinal fluids

*Material and methods:*

Egg-phosphatidylcholine (E-PC) was obtained from Lipoid GmbH (Ludwigshafen, Germany). Cholesterol and sodium taurocholate (minimum 95% TLC) were purchased from Sigma-Aldrich (Taufkirchen, Germany). Lime-bone (LB) gelatine was obtained from Gelita AG (Eberbach, Germany), Triton-X from Roth GmbH & Co KG (Karlsruhe, Germany) and 5(6)-carboxyfluorescein (CF) from Serva (Heidelberg, Germany). All other chemicals were obtained in the highest purity from the usual commercial sources.

E-PC and cholesterol were dissolved in chloroform/methanol 9:1 and then mixed to achieve an E-PC to cholesterol ratio of 60 to 40. Subsequently, the solvent was evaporated using a Rotavapor-R (Büchi Labortechnik AG, Flawil, Switzerland). The dried lipid mixture was weighed into a 2 ml Eppendorf cup and CF 50 mM in phosphate buffered saline (PBS) (pH 7.4, NaCl 135 mM, KCl 3 mM, $Na_2HPO_4$ 8 mM, $KH_2PO_4$ 1,5 mM) or in gelatine (10% (w/v) in bidistilled water) in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were further diluted with PBS in a 30 s mixing step directly after speed mixing to obtain liposomes with the desired final lipid concentration.
The non-encapsulated CF was separated from the liposomes by a Sephadex® G50 fine size exclusion chromatography. Release of the marker was determined at 37°C using a Fluoroskan Ascent (Thermo Fischer Scientific, Waltham, USA) after injection of the liposomes in Tris buffer pH 2 (Tris 50 mM, KCl 2.7 mM and NaCl 120 mM) or sodium taurocholate 11.11mM in PBS resulting in a 1:10 dilution of the formulations. Increase of fluorescence was measured at 485 nm excitation and 520 nm emission wavelength. Since the fluorescence of CF is pH-dependent, the samples were neutralized after 2, 10, 30 and 60 min with Tris buffer pH 10 to achieve a final pH of 7.4. The emission of the liposomes in the mixture of the two different Tris buffers was set as zero release control and the fluorescence in Triton-X 1% in the Tris buffer mix as 100% release control. The emission of CF in the other assay could be measured continuously and the emission in Triton-X 1% in PBS was set as 100% release. The emission in PBS was used as a negative control for the test in sodium taurocholate. All tests were performed in triplicate in Costar® 24 well plates (Corning, Kaiserslautern, Germany). In these type of wells the influence of the surface tension reduction on the fluorescence by the bile salt and Triton-X is less pronounced than in 96 well plates. The leakage of CF over the time was calculated as follows:

$$\% \text{ CF release} = \frac{FE - FE_0}{FE_{Trit} - FE_0} \, x \, 100\%$$

wherein FE is the fluorescence emission at the different time points, $FE_0$ is the emission of negative control and $FE_{Trit}$ the emission of liposomes after destruction with Triton-X 1%.

*Results:*

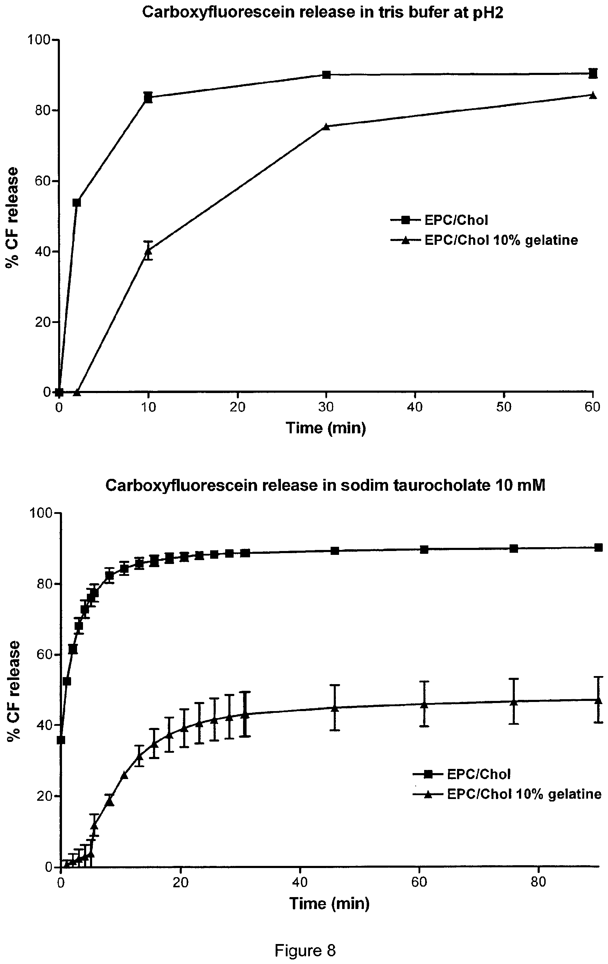

It can be seen that the addition of only 10% gelatine leads to a substantial higher stability under acidic conditions and against bile salts, making gelatine stabilized liposomes a suitable tool for oral delivery (Fig. 8).

Example 5:

Matrix liposomes containing human growth hormone

*Material and methods:*

Egg-phosphatidylcholine (E-PC) was obtained from Lipoid GmbH (Ludwigshafen, Germany). Cholesterol was purchased from Sigma-Aldrich (Taufkirchen, Germany). Lime-bone (LB) gelatine was obtained from Gelita AG (Eberbach, Germany) and Triton-X from Roth GmbH & Co KG (Karlsruhe, Germany). Human Growth Hormone (hGH) (Genotropin®) was obtained from Pfizer Pharma (Berlin, Germany). All other chemicals were obtained in the highest purity from the usual commercial sources.

E-PC and cholesterol were dissolved in chloroform/methanol 9:1 and then mixed to achieve an E-PC to cholesterol ratio of 50 to 50. Subsequently, the solvent was evaporated using a Rotavapor-R (Büchi Labortechnik AG, Flawil, Switzerland). The dried lipid mixture was weighed into a 2 ml Eppendorf cup and hGH (80 mg/ml) with gelatine (15% (w/v)) in bidistilled water in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were further diluted with PBS in a 30 s mixing step directly after speed mixing to obtain liposomes with the desired final lipid concentration.

Liposomes were diluted with PBS to an appropriate concentration and Z-average and polydispersity index (PDI) was determined using a Zetasizer® 3000 HS (Malvern, Works, UK) in the automatic mode.

The concentrated liposome suspension was applied to a glow discharged Quantifoil specimen support grid, blotted from one side in a humidified atmosphere for 4 sec using a Vitrobot (FEI, Hillsboro, OR, US), and plunged into liquid ethane. Grids were mounted under liquid nitrogen on a Gatan 3500 cold stage (Gatan, Munich, Germany). The stage was transferred on a Zeiss 923 (Sesam, Carl Zeiss SMT, Oberkochen, Germany) electron microscope equipped with a field emission gun operated at 200 kV and an in column corrected Omega filter with a slit width of 50 eV. Zeroloss Images were recorded with a 4k x 4k Tietz camera (Gauting, Germany) at about 10-20 m underfocus at a magnification of 50000 x.

200 $\mu$l of liposome dispersion were applied to a Sepharose® CL-4B column to separate non-encapsulated hGH. Liposomes were further diluted 1:10 with Triton-X 1% in PBS and un-columned vesicles as control were diluted 1:100 with Triton-X 1% in PBS. HGH concentration was determined by HPLC with a Dionex UltiMate® 3000 system (Dionex, Idstein, Germany) using an Acclaim® 120 C18 5-m column (4.6 mm x 250 mm) at 50°C and a UV PDA detector. Flow was kept constant during the run at 1 ml/min with 20% water plus 0.05% trifluoroacetic acid (TFA) and 80% acetonitrile plus 0.05% TFA as mobile phase. HGH concentration was determined at 218 nm against a calibration curve.

*Results:*

Main particle size of liposomes containing hGH and 15% gelatine was 212.8 nm ($\pm$ 9.48 nm), PDI 0.27 ($\pm$ 0.019) and encapsulation efficiency 60.3 % ($\pm$ 5.22%).

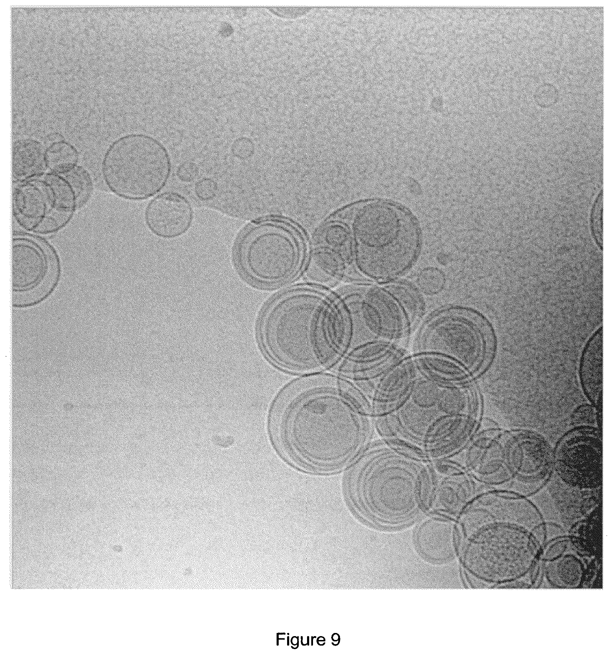

Size data and the TEM picture (Fig. 9) suggest the successful formation of liposomes containing gelatine and hGH after they were fully redispersed with PBS. The encapsulation efficiency of 60% is surprisingly high and shows that matrix liposomes have potential to be used for the oral delivery of protein drugs.

Example 6:

Stability of re-dispersed matrix liposomes

*Material and methods:*

Egg-phosphatidylcholine (E-PC) was obtained from Lipoid GmbH (Ludwigshafen, Germany). Cholesterol was purchased from Sigma-Aldrich (Taufkirchen, Germany). Lime-bone (LB) gelatine was obtained from Gelita AG (Eberbach, Germany). All other chemicals were obtained in the highest purity from the usual commercial sources.

E-PC and cholesterol were dissolved in chloroform/methanol 9:1 and then mixed to achieve an E-PC to cholesterol ratio of 60 to 40. Subsequently, the solvent was evaporated using a Rotavapor-R (Büchi Labortechnik AG, Flawil, Switzerland). The dried lipid mixture was weighed into a 2 ml Eppendorf cup and gelatine (10% and 20% (w/v) in bidistilled water) in a ratio of 2 to 3 (lipid to buffer) and glass beads in a ratio of 2 to 5 (lipid to glass beads) were added. The cups were mixed for 30 min at 3540 rpm in a Speedmixer (DAC 150 FVZ, Hauschild, Hamm, Germany) to form a vesicular phospholipid gel (VPG). Finally, the VPGs were either further diluted with PBS in a 30 s mixing step directly after speed mixing.

The liposome dispersions were stored in the fridge at 4 °C up to 3 years. Liposomes were diluted with PBS to an appropriate concentration and Z-average and poly-dispersity index (PDI) was determined directly after preparation and after 3 years of storage using a Zetasizer® 3000 HS (Malvern, Works, UK) in the automatic mode.

*Results:*

Main particle size of liposomes prepared with 10% gelatine was 141.8 nm ($\pm$ 4.21 nm) and with 20% gelatine 172.0 nm ($\pm$ 20.12 nm) directly after preparation. The Z-Average increased during the 3 years of storage for both types only less than 10 nm. The PDI was for both formulations directly after preparation below 0.25 and after 3 years still below 0.30. The results indicate an excellent storage stability of matrix liposomes.

**Claims**

1. A solid dosage form comprising:

   liposomes;
   water in an amount of between 5% and 95% (w/w), based on the total mass of the solid dosage form; and
   one or more solidifier(s);
   wherein said solidifier(s)
   form(s) a solid matrix in which said liposomes are embedded, and
   is/are contained in the inner lumen of said liposomes.

2. The solid dosage form of claim 1, wherein said liposomes comprise an agent selected from the group of pharmaceutically active agents and pro-forms thereof, diagnostic agents, nutritional supplements, and cosmetics.

**3.** The solid dosage form of claim 1 or claim 2, wherein the liposomes exhibit a Z-Average measured by dynamic light scattering after dilution in aqueous medium of at most 350 nm and a polydispersity index of at most 0.3.

**4.** The solid dosage form of any one of claims 1 to 3, wherein the liposomes are present as vesicular phospholipid gel (VPG).

**5.** The solid dosage form of any one of claims 1 to 4, wherein the solidifier(s) is/are selected from the group consisting of curable polymers, alginate, cellulose acetate phthalate, sodium carboxymethylcellulose, hydroxy ethylcellulose, hydroxy propylcellulose, methylcellulose, methylhydroxy ethylcellulose, polyacrylic acid and derivatives thereof, pectin, polyvinyl pyrrolidone (PVP), agarose, alginic acid, collagen, proteins, xanthan gum, carrageenan, tragacanth, chitosan, acacia, polyethylene glycol (PEG) having preferably a molecular weight from 1200 to 6000 Da, hydroxypropyl methylcellulose, pullulan, hydroxypropyl starch and gelatin.

**6.** The solid dosage form of claim 5, wherein the solidifier is gelatine and the content thereof in the solid dosage form is 1.5 to 25% (w/w) based on the total mass of the solid dosage form.

**7.** The solid dosage form of any one of claims 1 to 6, wherein the content of water in the solid dosage form is between 45% and 55% (w/w) based on the total mass of the solid dosage form.

**8.** The solid dosage form of any one of claims 2 to 7, wherein the agent is a pharmaceutically active agent selected from the group consisting of human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, or toxins, virus-derived vaccine antigens, octreotide, cyclosporine, rifampycin, lopinavir, ritonavir, vancomycin, telavancin, oritavancin, dalbavancin, bisphosphonates, itraconazole, danazol, paclitaxel, cyclosporin, naproxen, capsaicin, albuterol sulfate, terbutaline sulfate, diphenhydramine hydrochloride, chlorpheniramine maleate, loratidine hydrochloride, fexofenadine hydrochloride, phenylbutazone, nifedipine, carbamazepine, naproxen, cyclosporin, betamethosone, danazol, dexamethasone, prednisone, hydrocortisone, 17 beta-estradiol, ketoconazole, mefenamic acid, beclomethasone, alprazolam, midazolam, miconazole, ibuprofen, ketoprofen, prednisolone, methylprednisone, phenytoin, testosterone, flunisolide, diflunisal, budesonide, fluticasone, insulin, acylated insulin, glucagon-like peptide, acylated glucagon-like peptide, exenatide, lixisenatide, dulaglutide, liraglutide, albiglutide, taspoglutide, C-Peptide, erythropoietin, calcitonin, luteinizing hormone, prolactin, adrenocorticotropic hormone, leuprolide, interferon alpha-2b, interferon beta-la, sargramostim, aldesleukin, interferon alpha-2a, interferon alpha-n3alpha-proteinase inhibitor, etidronate, nafarelin, chorionic gonadotropin, prostaglandin E2, epoprostenol, acarbose, metformin, desmopressin, cyclodextrin, antibiotics, antifungal drugs, steroids, anticancer drugs, analgesics, anti-inflammatory agents, anthelmintics, anti-arrhythmic agents, penicillins, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, hypnotics, neuroleptics, astringents, beta-adrenoceptor blocking agents, blood products and substitutes, cardiacinotropic agents, contrast media, corticosteroids, cough suppressants, expectorants, mucolytics, diuretics, CNS-active compounds, dopaminergics, antiparkinsonian agents, hemostatics, immunological agents, lipid regulating agents, muscle relaxants, parasympathomimetics, parathyroid calcitonin, prostaglandins, radiopharmaceuticals, sex hormones, steroids, anti-allergic agents, stimulants, anoretics, sympathomimetics, thyroid agents, vasodilators, xanthines, heparins, therapeutic oligonucleotides, somatostatins and analogues thereof, and pharmacologically acceptable organic and inorganic salts or metal complexes thereof.

**9.** The solid dosage form of any one of claims 1 to 8 which is for oral or topical or parenteral administration.

**10.** A method for the production of the solid dosage form of any one of claims 1 to 9, said method comprising the step of:

(a) preparing liposomes in a dispersion comprising (i) water in an amount of between 5% and 95% (w/w), based on the total mass of the dispersion, and (ii) one or more solidifiers;
(b) forming the composition obtained in step (a) to a desired dosage form; and
(c) letting said composition solidify.

**11.** The method of claim 10, wherein the dispersion used in step (a) further comprises (iii) an agent selected from the group of pharmaceutically active agents and pro-forms thereof, diagnostic agents, nutritional supplements, and cosmetics.

**12.** The method of claim 10 or claim 11, wherein the liposomes are prepared by a method, selected from the group consisting of high pressure homogenization and dual asymmetric centrifugation (DAC).

**13.** The method of any one of claims 10 to 12, wherein the liposomes are prepared as vesicular phospholipid gel (VPG).

**14.** The method of any one of claims 10 to 13, wherein the solidifier is gelatine and the dispersion used in step (a) comprises said gelatine in an amount of 1.5 to 25% (w/w) based on the total mass of the dispersion.

**Patentansprüche**

**1.** Feste Darreichungsform, umfassend:

Liposomen;
Wasser in einer Menge zwischen 5% und 95% (w/w), bezogen auf die Gesamtmasse der festen Darreichungsform; und
einen oder mehrere Verfestiger;
wobei der/die Verfestiger
eine feste Matrix bilde(t/n), in welche die Liposomen eingebettet sind, und
im inneren Lumen der Liposomen enthalten ist/sind.

**2.** Feste Darreichungsform nach Anspruch 1, wobei die Liposomen ein Mittel umfassen, ausgewählt aus der Gruppe aus pharmazeutisch aktiven Mitteln und Vorstufen davon, diagnostischen Mitteln, Nahrungsergänzungsmitteln und Kosmetika.

**3.** Feste Darreichungsform nach Anspruch 1 oder Anspruch 2, wobei die Liposomen einen Z-Durchschnitt von höchstens 350 nm, gemessen durch dynamische Lichtstreuung nach Verdünnung in einem wässrigem Medium, und einen Polydispersitätsindex von höchstens 0,3 aufweisen.

**4.** Feste Darreichungsform nach einem der Ansprüche 1 bis 3, wobei die Liposomen als vesikuläres Phospholipidgel (VPG) vorliegen.

**5.** Feste Darreichungsform nach einem der Ansprüche 1 bis 4, wobei der/die Verfestiger ausgewählt ist/sind aus der Gruppe, bestehend aus vernetzbaren Polymeren, Alginat, Celluloseacetatphthalat, Natriumcarboxymethylcellulose, Hydroxyethylcellulose, Hydroxypropylcellulose, Methylcellulose, Methylhydroxyethylcellulose, Polyacrylsäure und Derivaten davon, Pektin, Polyvinylpyrrolidon (PVP), Agarose, Alginsäure, Kollagen, Proteinen, Xanthan, Carrageen, Tragant, Chitosan, Akazie, Polyethylenglykol (PEG), das vorzugsweise ein Molekulargewicht von 1200 bis 6000 Da aufweist, Hydroxypropylmethylcellulose, Pullulan, Hydroxypropylstärke und Gelatine.

**6.** Feste Darreichungsform nach Anspruch 5, wobei der Verfestiger Gelatine ist und deren Gehalt in der festen Darreichungsform 1,5 bis 25% (w/w), bezogen auf die Gesamtmasse der festen Darreichungsform, beträgt.

**7.** Feste Darreichungsform nach einem der Ansprüche 1 bis 6, wobei der Wassergehalt in der festen Darreichungsform zwischen 45% und 55% (w/w), bezogen auf die Gesamtmasse der festen Darreichungsform, beträgt.

**8.** Feste Darreichungsform nach einem der Ansprüche 2 bis 7, wobei das Mittel ein pharmazeutisch aktives Mittel ist, ausgewählt aus der Gruppe, bestehend aus menschlichem Wachstumshormon, Wachstumshormon-freisetzendem Hormon, Wachstumshormon-freisetzendem Peptid, Interferonen, koloniestimulierenden Faktoren, Interleukinen, Makrophagen-aktivierendem Faktor, Makrophagen-Peptid, B-Zellfaktor, T-Zellfaktor, Protein A, Allergie-Inhibitor, Zell-Nekrose-Glykoproteinen, Immunotoxin, Lymphotoxin, Tumor-Nekrose-Faktor, Tumor-Suppressoren, Metastasen-Wachstumsfaktor, alpha-1 Antitrypsin, Albumin und Polypeptidfragmenten davon, Apolipoprotein-E, Erythropoietin, Faktor VII, Faktor VIII, Faktor IX, Plasminogen-aktivierendem Faktor, Urokinase, Streptokinase, Protein C, C-reaktivem Protein, Renin-Inhibitor, Kollagenase-Inhibitor, Superoxid-Dismutase, Thrombozyten-Wachstumsfaktor, epidermalem Wachstumsfaktor, osteogenem Wachstumsfaktor, knochenstimulierendem Protein, Calcitonin, Insulin, Atriopeptin, knorpelinduzierendem Faktor, bindegewebsaktivierendem Faktor, follikelstimulierendem Hormon, luteinisierendem Hormon, luteinisierendes Hormon-Releasing-Hormon, Nervenwachstumsfaktoren, Nebenschilddrüsenhormon, Relaxin, Sekretin, Somatomedin, insulinähnlichem Wachstumsfaktor, Nebennierenrindenhormon, Glucagon, Cholecystokinin, Pankreas-Polypeptid, gastrinfreisetzendem Peptid, Corticotropin-Releasing-Faktor, schilddrüsenstimulierendem Hormon, monoklonalen oder polyklonalen Antikörpern gegen verschiedene Viren, Bakterien oder Toxine, Virusabgeleiteten Impfstoff-Antigenen, Octreotid, Cyclosporin, Rifampycin, Lopinavir, Ritonavir, Vancomycin, Telavancin, Oritavancin, Dalbavancin, Bisphosphonaten, Itraconazol, Danazol, Paclitaxel, Cyclosporin, Naproxen, Capsaicin, Albuterolsulfat, Terbutalinsulfat, Diphenhydraminhydrochlorid, Chlorpheniraminmaleat, Loratidinhydrochlorid, Fexofenadinhydrochlorid, Phenylbutazon, Nifedipin, Carbamazepin, Naproxen, Cyclosporin, Betamethoson, Danazol, Dexamethason, Prednison, Hydrocortison, 17-beta-Estradiol, Ketoconazol, Mefenaminsäure, Beclomethason, Alprazolam, Midazolam, Miconazol, Ibuprofen, Ketoprofen, Prednisolon, Methylprednison, Phenytoin, Testosteron, Flunisolid, Diflunisal, Budesonid, Fluticason, Insulin, acyliertem Insulin, glucagonähnlichem Peptid, acyliertem glucagonähnlichem Peptid, Exenatid, Lixisenatid, Dulaglutid, Liraglutid, Albiglutid, Taspoglutid, C-Peptid, Erythropoietin, Calcitonin, luteinisierendem Hormon, Prolaktin, adrenocorticotropem Hormon, Leuprolid, Interferon alpha-2b, Interferon beta-la, Sargramostim, Aldesleukin, Interferon alpha-2a, Interferon alpha-n3alpha-Proteinase-Hemmer, Etidronat, Nafarelin, Choriongonadotropin, Prostaglandin E2, Epoprostenol, Acarbose, Metformin, Desmopressin, Cyclodextrin, Antibiotika, Antimykotika, Steroiden, Krebsmedikamenten, Analgetika, entzündungshemmenden Mitteln, Anthelmintika, Antiarrhythmika, Penicillinen, Antikoagulantien, Antidepressiva, Antidiabetika, Antiepileptika, Antihistaminika, Antihypertonika, Antimuskarinika, antimycobakteriellen Mitteln, Antineoplastika, Immunsuppressiva, Antithyroidika, antiviralen Mitteln, anxiolytischen Beruhigungsmitteln, Hypnotika, Neuroleptika, Adstringens, beta-Adrenozeptor-Blockern, Blutprodukten und -ersatzstoffen, Herz-Kreislaufmitteln, Kontrastmitteln, Kortikosteroiden, Hustenblockern, Schleimlösern, Mukolytika, Diuretika, ZNS-Wirkstoffen, Dopaminergika, Antiparkinsonmitteln, Hämostatika, immunologischen Mitteln, lipidregulierenden Mitteln, Muskelrelaxantien, Parasympathomimetika, Nebenschilddrüsen-Calcitonin, Prostaglandinen, Radiopharmazeutika, Sexualhormonen, Steroiden, Antiallergika, Stimulantien, Anoretika, Sympathomimetika, Schilddrüsenmitteln, Vasodilatatoren, Xanthinen, Heparinen, therapeutischen Oligonukleotiden, Somatostatinen und Analoga davon sowie pharmakologisch verträglichen organischen und anorganischen Salzen oder Metallkomplexen davon.

9. Feste Darreichungsform nach einem der Ansprüche 1 bis 8, die für orale oder topische oder parenterale Verabreichung vorgesehen sind.

10. Verfahren für die Herstellung der festen Darreichungsform nach einem der Ansprüche 1 bis 9, wobei das Verfahren den Schritt umfasst:

   (a) Herstellen von Liposomen in einer Dispersion, umfassend (i) Wasser in einer Menge zwischen 5% und 95% (w/w), bezogen auf die Gesamtmasse der Dispersion, und (ii) einen oder mehrere Verfestiger;
   (b) Formen der in Schritt (a) erhaltenen Zusammensetzung zu einer gewünschten Darreichungsform; und
   (c) Verfestigenlassen der Zusammensetzung.

11. Verfahren nach Anspruch 10, wobei die in Schritt (a) verwendete Dispersion weiter (iii) ein Mittel, ausgewählt aus der Gruppe aus pharmazeutisch aktiven Mitteln und Vorstufen davon, diagnostischen Mitteln, Nahrungsergänzungsmitteln und Kosmetika, umfasst.

12. Verfahren nach Anspruch 10 oder Anspruch 11, wobei die Liposomen durch ein Verfahren hergestellt werden, ausgewählt aus der Gruppe, bestehend aus Hochdruckhomogenisierung und doppelter asymmetrischer Zentrifugation (DAC).

13. Verfahren nach einem der Ansprüche 10 bis 12, wobei die Liposomen als vesikuläres Phospholipidgel (VPG) hergestellt werden.

14. Verfahren nach einem der Ansprüche 10 bis 13, wobei der Verfestiger Gelatine ist und die in Schritt (a) verwendete Dispersion die Gelatine in einer Menge von 1,5 bis 25% (w/w), bezogen auf die Gesamtmasse der Dispersion, enthält.

**Revendications**

1. Présentation médicamenteuse solide comprenant :

   des liposomes ;
   de l'eau en une quantité allant de 5% à 95% (p/p), sur base de la masse totale de la présentation médicamenteuse solide, et
   un ou plusieurs solidifiant(s) ;
   dans laquelle ledit/lesdits solidifiant(s) forme(nt)une matrice solide dans laquelle lesdits liposomes sont incorporés, et il(s) est/sont contenus dans la lumière interne desdits liposomes.

2. Présentation médicamenteuse solide selon la revendication 1, dans laquelle lesdits liposomes comprennent un agent choisi parmi le groupe des agents pharmaceutiquement actifs et de leurs précurseurs, les agents diagnostiques, les compléments nutritionnels et les cosmétiques.

3. Présentation médicamenteuse solide selon la revendication 1 ou 2, dans laquelle les liposomes présentent une moyenne Z mesurée par diffusion dynamique de la lumière après dilution dans un milieu aqueux d'au plus 350 nm et un indice de polydispersité d'au plus 0,3.

4. Présentation médicamenteuse solide selon l'une quelconque des revendications 1 à 3, dans laquelle les liposomes sont présents sous forme d'un gel phospholipidique vésiculaire (VPG).

5. Présentation médicamenteuse solide selon l'une quelconque des revendications 1 à 4, dans laquelle le ou les solidifiants sont choisis parmi le groupe consistant en les polymères durcissables, l'alginate, l'acétophtalate de cellulose, la carboxyméthylcellulose sodique, l'hydroxyéthylcellulose, l'hydroxypropylcellulose, la méthylcellulose, la méthylhydroxyéthylcellulose, le poly(acide acrylique) et ses dérivés, la pectine, la polyvinylpyrrolidone (PVP), l'agarose, l'acide alginique, le collagène, des protéines, la gomme de xanthane, le carraghénane, la gomme adragante, le chitosane, la gomme arabique, un polyéthylèneglycol (PEG) ayant de préférence un poids moléculaire allant de 1200 à 6000 Da, l'hydroxypropylméthylcellulose, le pullulane, l'hydroxypropylamidon et la gélatine.

6. Présentation médicamenteuse solide selon la revendication 5, dans laquelle le solidifiant est la gélatine et sa teneur dans la présentation solide se situe dans l'intervalle allant de 1,5 à 25% (p/p) sur base de la masse totale de la présentation solide.

7. Présentation médicamenteuse solide selon l'une quelconque des revendications 1 à 6, dans laquelle la teneur en eau dans la présentation solide se situe dans l'intervalle allant de 45% à 55% (p/p) sur base de la masse totale de la présentation solide.

8. Présentation médicamenteuse solide selon l'une quelconque des revendications 2 à 7, dans laquelle l'agent est un agent pharmaceutiquement actif choisi parmi le groupe consistant en l'hormone de croissance humaine, une hormone libérant l'hormone de croissance, un peptide libérant l'hormone de croissance, des interférons, des facteurs de stimulation des colonies, des interleukines, le facteur d'activation des macrophages, le peptide de macrophage, le facteur des lymphocytes B, le facteur des lymphocytes T, la protéine A, un inhibiteur d'allergie, des glycoprotéines de nécrose cellulaire, une immunotoxine, une lymphotoxine, le facteur de nécrose tumorale, des suppresseurs tumoraux, le facteur de croissance des métastases, l'antitrypsine alpha-1, l'albumine et des fragments polypeptidiques de celle-ci, l'apolipoprotéine E, l'érythropoïétine, le facteur VII, le facteur VIII, le facteur IX, le facteur d'activation du plasminogène, l'urokinase, la streptokinase, la protéine C, la protéine C réactive, un inhibiteur de rénine, un inhibiteur de collagénase, la superoxyde dismutase, le facteur de croissance dérivé des plaquettes, le facteur de croissance épidermique, le facteur de croissance ostéogénique, la protéine de stimulation osseuse, la calcitonine, l'insuline, l'atriopeptine, le facteur d'induction du cartilage, le facteur d'activation du tissu conjonctif, l'hormone de stilumation folliculaire, l'hormone lutéinisante, une hormone libérant l'hormone lutéinisante, des facteurs de croissance nerveuse, l'hormone parathyroïdinenne, la relaxine, la secrétine, la somatomédine, le facteur de croissance de type insuline, l'hormone corticosurrénale, le glucagon, le cholécystokinine, le polypeptide pancréatique, le peptide libérant la gastrine, le facteur libérant la corticotropine, l'hormone de stimulation thyroïdienne, des anticorps monoclonaux ou polyclonaux contre différents virus, bactéries ou toxines, des antigènes vaccinaux dérivés de virus, l'octréotide, la cyclosporine, la rifampicine, le lopinavir, le ritonavir, la vancomycine, la télavancine, l'oritavancine, la dalbavancine, des bisphosphonates, l'itraconazole, le danazole, le paclitaxel, la cyclosporine, le naproxène, la capsaïcine, le sulfate d'albutérol, le sulfate de terbutaline, le chlorhydrate de diphénhydramine, le maléate de chlorphéniramine, le chlorhydrate de loratidine, le chlorhydrate de fexofénadine, la phénylbutazone, la nifédipine, la carbamazépine, le naproxène, la cyclosporine, la bêtaméthasone, le danazole, la dexaméthasone, la prednisone, l'hydrocortisone, le 17-bêta-estradiol, le kétoconazole, l'acide meténamique, le béclométhasone, l'alprazolam, le midazolam, le miconazole, l'ibuprofène, le kétoprofène, la prednisolone, la méthylprednisolone, la phénytoïne, la testostérone, le flunisolide, le diflunisal, le budésonide, la fluticasone, l'insuline, l'insuline acétylée, le peptide de type glucagon, le peptide de type glucagon acétylé, l'exénatide, le lixisénatide, le dulaglutide, le liraglutide, l'albiglutide, le taspoglutide, le peptide C, l'érythropoïétine, la calcitonine, l'hormone lutéinisante, la prolactine, l'hormone adrénocorticotropique, le leuprolide, l'interféron alpha-2b, l'interféron bêta-1a, le sargramostim, l'aldesleukine, l'interféron alpha-2a, l'interféron alpha-n3, l'inhibiteur d'alpha-protéinase, l'étidronate, la nafaréline, la gonadotropine chorionique, la prostaglandine E2, l'époprosténol, l'acarbose, la metformine, la desmopressine, une cyclodextrine, des antibiotiques, des médicaments antifongiques, des stéroïdes, des médicaments anticancéreux, des analgésiques, des agents anti-inflammatoires, des anthelminthiques, des agents antiarythmiques, des pénicillines, des anticoagulants, des antidépresseurs, des agents antidiabétiques, des antiépileptiques, des antihistaminiques, des agents antihypertenseurs, des agents antimuscariniques, des agents antimycobactériens, des agents antinéoplasiques, des immunosuppresseurs, des agents antithyroïdiens, des agents antiviraux, des sédatifs anxiolytiques, des hypnotiques, des neuroleptiques, des astringents, des agents de blocage du bêta-adrénorécepteur, des produits et substituts sanguins, des agents cardiacinotropiques, des agents de contraste, des corticostéroïdes, des antitussifs, des expectorants, des mucolytiques, des diurétiques, des composés actifs sur le SNC, des dopaminergiques, des agents antiparkinsoniens, des hémostatiques, des agents immunologiques, des agents de régulation lipidique, des relaxants musculaires, des parasympathicomimétiques, la calcitonine parathyroïde, des prostaglandines, des produits radiopharmaceutiques, des hormones sexuelles, des stéroïdes, des agents antiallergiques, des stimulants, des anorexigènes, des sympathicomimétiques, des agents thyroïdiens, des vasodilatateurs, des xanthines, des héparines, des oligonucléotides thérapeutiques, des somatostatines et leurs analogues, et leurs sels organiques et inorganiques pharmacologiquement acceptables ou leurs complexes métalliques.

9. Présentation médicamenteuse solide selon l'une quelconque des revendications 1 à 8, qui est destinée à une administration orale, topique ou parentérale.

10. Procédé de préparation de la présentation médicamenteuse solide selon l'une quelconque des revendications 1 à 9, le procédé comprenant les étapes de :

   (a) préparation des liposomes dans une dispersion comprenant (i) de l'eau en une quantité allant de 5% à 95% (p/p), sur base de la masse totale de la dispersion, et (ii) un ou plusieurs solidifiant(s) ;
   (b) mise en forme de la composition obtenue à l'étape (a) en une forme posologique souhaitée ; et
   (c) solidification de la composition.

11. Procédé selon la revendication 10, dans lequel la dispersion utilisée à l'étape (a) comprend en outre, (iii) un agent choisi parmi le groupe des agents pharmaceutiquement actifs et de leurs précurseurs, les agents diagnostiques, les compléments nutritionnels et les cosmétiques

12. Procédé selon la revendication 10 ou 11, dans lequel les liposomes sont préparés par un procédé, choisi parmi le groupe consistant en une homogénéisation à haute pression et la centrifugation asymétrique double (DAC).

13. Procédé selon l'une quelconque des revendications 10 à 12, dans lequel les liposomes sont préparés sous forme de gel phospholipidique vésiculaire (VPG).

14. Procédé selon l'une quelconque des revendications 10 à 13, dans lequel le solidifiant est la gélatine et la dispersion utilisée à l'étape (a) comprend ladite gélatine en une quantité allant de 1,5 à 25% (p/p) sur base de la masse totale de la dispersion.

Figure 1

## Size gel%

## PDI

Figure 2

Figure 3

## Size

## PDI

Figure 4

Figure 5

## EE% Matrixliposomen with CF solidified

## EE% Matrixliposomen with FITC solidified

Figure 6

## EE% ML with CF direct vs solidified

## EE% ML with FITC direct vs. solidified

Figure 7

**Carboxyfluorescein release in tris bufer at pH2**

**Carboxyfluorescein release in sodim taurocholate 10 mM**

Figure 8

Figure 9

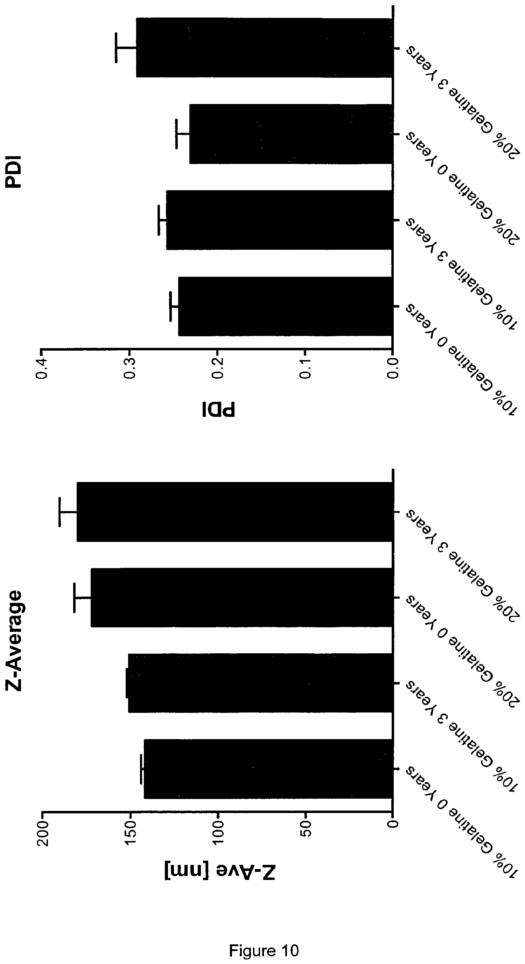

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6399094 B1, M. Brandl
- EP 1674081 A1, U. Massing
- US 20100239654 A1
- EP 0393049 B1, J. Hauton
- WO 0393049 A
- US 2011020428 A1
- US 4839111 A
- WO 2006103657 A2, A. Pinhasi and M. Gomberg
- WO 2004009053 A2, M. Farber and J. Farber
- US 20080279921 A1, V. Albrecht and D. Scheglmann

### Non-patent literature cited in the description

- **G. FRICKER et al.** *J. Pept. Sci.,* 1996, vol. 2 (4), 195-211
- **H. SOHI et al.** *Drug. Dev. Ind. Pharm.,* 2010, vol. 36 (3), 254-282
- **R. SINGH et al.** *J. Pharm. Sci.,* 2008, vol. 97 (7), 2497-2523
- **M. SHIMIZU.** *Nahrung,* 1999, vol. 43 (3), 154-158
- **J. HAMMAN et al.** *BioDrugs,* 2006, vol. 19 (3), 165-177
- **V. TANG et al.** *Biophys. J.,* 2003, vol. 84 (3), 1660-1673
- **L. R. BROWN.** *Exp. Opin. Drug Del.,* 2005, vol. 2 (1), 29-42
- **C. LIPINSKI et al.** *Adv. Drug Deliver. Rev.,* 1997, vol. 23 (1-3), 3-25
- **E. ZIV et al.** *Microsc. Res. Tech.,* 2000, vol. 49 (4), 346-52
- **A. D. BANGHAM et al.** *J. Mol. Biol.,* 1965, vol. 13 (1), 238-52
- **A. JESORKA et al.** *Annu. Rev. Anal. Chem.,* 2008, vol. 1, 801-832
- **F. SZOKA et al.** *Annu. Rev. Biophys. Bioeng.,* 1980, vol. 9, 467-508
- **T. LIAN et al.** *J. Pharm. Sci.,* 2001, vol. 90 (6), 667-680
- **B. MUI et al.** *Meth. Enzymol.,* 2003, vol. 367, 3-14
- **E. C. VAN WINDEN.** *Meth. Enzymol.,* 2003, vol. 367, 99-110
- **MASSING et al.** Dual asymmetric centrifugation (DAC)-A new technique for liposome preparation. *J Control Release,* 2008, vol. 125 (1), 16-24
- **CROWE et al.** Is Vitrification Sufficient to Preserve Liposomes during Freeze-Drying?. *Cryobiology,* 1994, vol. 31 (4), 355-366